# EUROPEAN PATENT APPLICATION

(11) **EP 3 533 445 A1**
(43) Date of publication of application: **04.09.2019**
(21) Application number: 19151241.7
(22) Date of filing: 18.03.2010
(51) Int. Cl.: A61K 31/21, A61K 31/35, A61K 38/22, A61K 31/557, A61P 7/00, A61K 38/16

(54) **COMPOSITIONS COMPRISING CYCLIC AMP ENHANCERS AND/OR EP LIGANDS, AND METHODS OF PREPARING AND USING THE SAME**

(30) Priority: 19.03.2009 US 16171709 P; 05.10.2009 US 24876509 P
(62) Divisional of application: 10754130.2
(71) Applicant: Fate Therapeutics, Inc., La Jolla, California 92037 (US)
(72) Inventor: MENDLEIN, John, D, Leucadia, CA California 92024 (US); FAROUZ, Francine, S, San Carlos CA 94070 (US); THIES, R, Scott, Seatle WA 98199 (US); SHOEMAKER, Daniel, San Diego, CA California 92130 (US)
(74) Representative: Donald, Jenny Susan

(57) **Abstract**

Provided are improved pharmaceutical compositions that comprise ligands or agonists to prostaglandin EP receptors, and/or cyclic AMP enhancers, and suitable organic solvents that are substantially free of methyl acetate, the compositions being provided for storage and/or use in an endotoxin-free vessel, such as a tube or PE bag. The compositions are suitable for in vitro, ex vivo, and in vivo use, and in particular for ex vivo therapeutic use, such as in hematopoietic stem cell transplants. Also provided are methods of using the compositions in ex vivo therapeutic applications, and methods of preparing the compositions. Kits with instructions on use are also provided.

## Description

### CROSS REFERENCE TO RELATED APPLICATIONS

This application claims the benefit under 35 U.S.C. § 119(e) of U.S. Provisional Application No. 61/161,717, filed March 19, 2009, and U.S. Provisional Application No. 61/248,765, filed October 5, 2009, each of which is incorporated by reference in its entirety.

### FIELD OF THE INVENTION

The present invention relates to improved pharmaceutical compositions comprising cyclic AMP (cAMP) enhancers and/or ligands to prostaglandin EP receptors in a suitable organic solvent and contained within a sterile and endotoxin free vessel, and to methods of preparing and using the same. These pharmaceutical compositions are mainly for use in *ex vivo* therapeutic applications, such as in stem cell transplant therapies, but can be used in other ways that will be apparent to persons skilled in the art.

### DESCRIPTION OF THE RELATED ART

Prostaglandins, such as prostaglandin E₂ (PGE₂), analogs thereof, and other ligands to prostaglandin EP receptors, as well as cAMP enhancers, show potential for use in *ex vivo* therapeutic uses, among other uses. For instance, it has been shown that PGE₂ and other ligands to EP receptors are capable of stimulating the growth and expansion of hematopoietic stem cells (HSCs) prior to, during, and/or subsequent to cell transplant procedures *(see, e.g.,* WO 2008/073748, herein incorporated by reference in its entirety). Given the well appreciated role of stem cell-related therapies in the treatment of a variety of pathological or malignant conditions, properly formulated compositions of ligands to prostaglandin EP receptors, as well as properly formulated compositions of cAMP enhancers, likewise have great potential in the treatment of such conditions.

However, despite their potential, PGE₂ analogs and other ligands to prostaglandin EP receptors have not been used to any large extent for *ex vivo* treatments. Among other potential concerns, such agents have problematic *in vivo* characteristics, such as by undergoing rapid oxidation (*in vivo*) to form inactive or toxic metabolites. In addition, the aqueous stability of many prostanoid ligands is typically less than 12 hours, which often leads to a dehydration event *(e.g.,* the formation of 16, 16 PGA-2, which is inactive) in water at neutral pH, including about pH 6.0 to about pH 8.0. Their low aqueous solubility creates other difficulties in therapeutic uses, as certain organic solvents may have undesired effects on cells in *ex vivo* treatments.

The melting temperature of such EP ligands also creates problems, causing the formation of a non-crystalline compound ("oil") at room temperature. Certain of these compounds are unstable as an oil, and may undergo isomerization from *cis* to *trans* forms, and may also undergo epimerization. These oils are also viscous, making them difficult to handle.

While some PGE ligands have been used for clinical purposes, none have been used for *ex vivo* purposes. Therefore, there is an need for articles of manufacture and compositions to improve or facilitate the action of these types of compounds for *ex vivo* and *in vivo* treatments for humans, especially for HSC, pluripotent cell, or multi-potent cell transplant-related therapies, and for methods of preparing and using the same.

### BRIEF SUMMARY

Embodiments of the present invention relate generally to compositions, and methods of use and preparation thereof, comprising an agent selected from a cyclic AMP (cAMP) enhancer and a ligand to a prostaglandin EP receptor, and an organic solvent, wherein the agent and the organic solvent are contained within a sterile and endotoxin free vessel, and wherein the composition is suitable for *ex vivo* administration to mammalian (e.g., human) cells.

In certain embodiments, the agent is a cAMP enhancer. In certain embodiments, the cAMP enhancer is selected from dibutyryl cAMP (DBcAMP), phorbol ester, forskolin, sclareline, 8-bromo-cAMP, cholera toxin (CTx), aminophylline, 2,4 dinitrophenol (DNP), norepinephrine, epinephrine, isoproterenol, isobutylmethylxanthine (IBMX), caffeine, theophylline (dimethylxanthine), dopamine, rolipram, iloprost, prostaglandin E₁, prostaglandin E₂, pituitary adenylate cyclase activating polypeptide (PACAP), and vasoactive intestinal polypeptide (VIP).

In certain embodiments, the agent is a ligand to a prostaglandin receptor, including wherein the ligand is a prostaglandin EP receptor agonist. In certain embodiments, the ligand is prostaglandin E₂ (PGE₂), or an analog thereof. In certain embodiments, the PGE₂ analog is selected from 16,16-dimethyl PGE₂, 16-16 dimethyl PGE₂ p-(p-acetamidobenzamido) phenyl ester, 11-deoxy-16,16-dimethyl PGE₂, 9-deoxy-9-methylene-16, 16-dimethyl PGE₂, 9-deoxy-9-methylene PGE₂, 9-keto Fluprostenol, 5-trans PGE₂, 17-phenyl-omega-trinor PGE₂, PGE₂ serinol amide, PGE₂ methyl ester, 16-phenyl tetranor PGE₂, 15(S)-15-methyl PGE₂, 15(R)-15-methyl PGE₂, 8-iso-15-keto PGE₂, 8-iso PGE₂ isopropyl ester, 20-hydroxy PGE₂, 11-deoxy PGE₁, nocloprost, sulprostone, butaprost, 15-keto PGE₂, and 19 (R) hydroxyy PGE₂. In certain specific embodiments, the PGE₂ analog is 16,16-dimethyl PGE₂.

In certain embodiments, the ligand is a non-PGE₂-based ligand. In certain embodiments, the non-PGE₂-based ligand is selected from the group consisting of an EP₁ agonist, an EP₂ agonist, an EP₃ agonist, and an EP₄ agonist. In certain embodiments, the non-PGE₂-based EP₁ agonist is selected from ONO-DI-004 and ONO-8713. In certain embodiments, the non-PGE₂-based EP₂ agonist is selected from CAY10399, ONO_8815Ly, ONO-AE1-259, and CP-533,536. In certain embodiments, the non-PGE₂-based EP₃ agonist is selected from AE5-599, MB28767, GR 63799X, ONO-NT012, and ONO-AE-248. In certain embodiments, the non-PGE₂-based EP₄ agonist is selected from ONO-4819, APS-999 Na, AH23848, and ONO-AE1-329. In certain embodiments, the prostaglandin EP receptor is selected from EP₁, EP₂, EP₃, and EP₄.

In certain embodiments, the agent is produced by good manufacturing practice (GMP). In certain embodiments, the agent is at least 90%, 95%, or 98% pure by high pressure liquid chromatography (HPLC). In a preferred embodiment, the agent is 16,16-dimethyl PGE₂ that is at least 90%, 95%, or 98% pure by HPLC.

In certain embodiments, the organic solvent is substantially free of methyl acetate. In certain embodiments, the organic solvent is selected from the group consisting of dimethyl sulfoxide (DMSO), N,N-dimethylformamide (DMF), dimethoxyethane (DME), dimethylacetamide, and combinations thereof. In certain specific embodiments, the organic solvent is DMSO.

In certain embodiments, the agent is present in the solvent at a concentration of about 100nM to about 10mM, or about 1mM to about 10mM, or about 100nM to about 1µM. In certain embodiments, the agent is present at a concentration of about 10mM.

In certain embodiments, the claimed compositions may comprise an inert gas in the vessel. Certain embodiments may comprise an air overlay in the vessel. In certain embodiments, the vessel is a bag, capsule, vial, tube, dish, or syringe that is suitable for storage of the composition. In certain embodiments, the vessel is a single use vessel.

In certain embodiments, the vessel is a bag, vial, tube, dish, or syringe that further comprises cord blood or human cells in a suitable medium, and wherein the vessel is suitable for *ex vivo* treatment of the cells. In certain embodiments, the organic solvent volume is less than about 1% of the total volume of the suitable medium. In certain embodiments, the organic solvent volume is less than about 0.1% of the total volume of the suitable medium. In certain embodiments, the human cells comprise hematopoietic stem cells.

In certain particular embodiments, the agent is 16,16 dimethyl PGE₂, preferably 16,16 dimethyl PGE₂ that is at least 90%, 95%, or 98% pure by HPLC, at a final concentration of about 10mM, the organic solvent is dimethyl sulfoxide (DMSO) that is substantially free of methyl acetate, the vessel is a 2ml vial with a teflon coated stopper, cap, or lid, and there is an air overlay in the vial.

Certain embodiments relate generally to methods of preparing a composition suitable for *ex vivo* administration to mammalian cells, comprising (a) reducing the volume of a first composition in a vessel that comprises methyl acetate and an agent selected from a cyclic AMP (cAMP) enhancer and a ligand to a prostaglandin EP receptor, to create a second composition, wherein the second composition is substantially free of the methyl acetate; and (b) adding an organic solvent to the second composition in the vessel, wherein the organic solvent is not methyl acetate and is suitable for *ex vivo* administration to mammalian cells, thereby preparing the composition suitable for *ex vivo* administration to mammalian cells. In certain embodiments, reducing the volume of the first composition in (a) comprises evaporating the methyl acetate.

In certain embodiments of the method of the invention, the agent, the organic solvent, and the vessel are each independently selected from agents, organic solvents, and vessels of the invention as described herein.

In certain embodiments, the agent is present in the organic solvent at a final concentration of about 100nM to about 10mM, or about 1mM to about 10mM, or about 100nM to about 1µM. In certain particular embodiments, the agent is present in the organic solvent at a concentration of about 10mM.

In certain embodiments, the agent is produced by good manufacturing practice (GMP). In certain embodiments, the agent is at least 90%, 95%, or 98% pure by high pressure liquid chromatography (HPLC).

In certain embodiments of the method of the invention, the agent is 16,16 dimethyl PGE₂ at final concentration of about 10mM, the organic solvent of step (b) is dimethyl sulfoxide (DMSO) that is substantially free of methyl acetate, the vessel is a 2ml vial with a teflon lid, stopper, or cap, and there is an air overlay in the vial.

Certain of the above methods may further comprise step (c) transferring the composition from the vessel to a second vessel, wherein the second vessel is endotoxin free and is suitable for storage or *ex vivo* administration of the composition. In certain embodiments, the second vessel is a bag, capsule, vial, tube, dish, or syringe. In certain embodiments, the second vessel is a single use vessel.

Also, certain of the above methods may further comprise step (c) transferring the composition from the vessel to a second vessel, wherein the second vessel is 2ml vial with a teflon cap that is endotoxin free and is suitable for storage or *ex vivo* administration of the composition, wherein the agent is 16,16 dimethyl PGE₂ at final concentration of about 10mM, wherein the organic solvent of step (b) is dimethyl sulfoxide (DMSO) that is substantially free of methyl acetate, and wherein there is an air overlay in the vial.

Also, certain of the above methods may further comprise step (c) transferring the composition from the vessel to a second vessel, wherein the second vessel is suitable for *ex vivo* treatment conditions and comprises cord blood. Or, certain of the above methods may further comprise step (c) transferring the composition to a second vessel, wherein the second vessel is suitable for *ex vivo* treatment conditions and comprises human cells in a suitable medium.

In certain embodiments, the organic solvent volume is less than about 1% of the total volume of the suitable medium. In certain embodiments, the organic solvent volume is less than about 0.1% of the total volume of the suitable medium. In certain embodiments, the human cells comprise hematopoietic stem cells (HSCs).

Certain embodiments include methods of stimulating hematopoietic stem cell (HSC) growth or expansion, comprising transferring a composition of the present invention to a second vessel that is suitable for *ex vivo* treatment conditions, wherein the second vessel comprises cord blood or HSCs in suitable medium, thereby stimulating HSC growth or expansion. Such embodiments may further comprise administering the HSCs to a subject.

Certain embodiments may comprise a kit, comprising a composition of claim 1 and instructions on use of the composition for *ex vivo* administration to mammalian cells. In certain embodiments, the *ex vivo* administration to mammalian cells comprises *ex vivo* therapeutic use in humans.

### BRIEF DESCRIPTION OF THE DRAWINGS

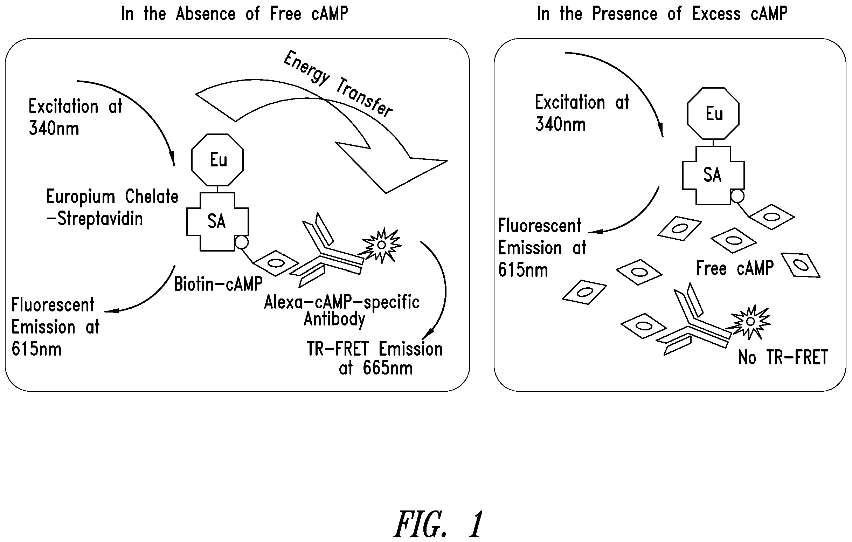

Figure 1 illustrates a fluorescence resonance energy transfer time-resolved (FRET) assay that may be used to measure the potency of a cAMP enhancer.

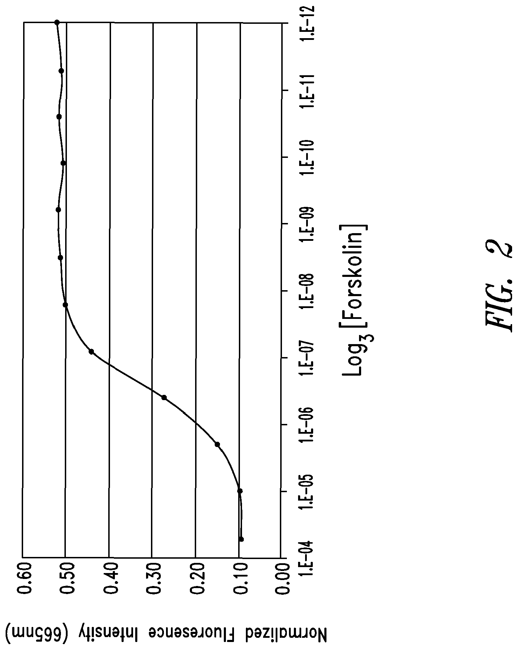

Figure 2 shows a dose response curve for LS142T treated with forskolin, measured according to the TR-FRET assay of Example 2. In this Figure, a dose response was observed, in which increasing concentrations of the forskolin (from right to left) caused an increase in cytosolic cAMP, as indicated by reduced fluorescence emission of the fluorescein-tagged antibody. ID₅₀ values could then be calculated from this data to compare the relative potencies of different cAMP enhancers.

### DETAILED DESCRIPTION

Embodiments of the present invention relate generally to the discovery of improved compositions of prostaglandin EP ligands and cAMP enhancers (*i.e*., "agents," as used herein), having desirable storage, handling, and physico-chemical properties, and which are suitable for many research and therapeutic uses, especially *ex vivo* therapeutic uses. Embodiments of the present invention also relate to methods of preparing compositions comprising such agents, as well as methods of using the compositions in therapeutic applications, such as *in vivo* and *ex vivo* therapeutic applications involving the isolation, *in vivo* or *ex vivo* expansion, and subsequent administration of hematopoietic stem cells to a subject in need thereof.

Generally, as detailed below, the improved compositions of the present invention relate, in pertinent part, to the identification and use of suitable organic solvents for the prostaglandin EP ligands and cAMP enhancers of the invention, such as dimethyl sulfoxide (DMSO), as well as the identification and use of optimal storage and working concentrations in such solvents to enhance stability and biological activity with regard to the contemplated uses. The improved, highly purified compositions of the present invention may also be produced by good manufacturing practice, and to facilitate their subsequent therapeutic use are typically stored and used in sterile and endotoxin-free vessels, such as a 2ml tube with a teflon coated cap, lid or stopper, or a medical grade polyethylene (PE) bag that is suitable for incubation of the compositions with various sources of cells *(e.g.,* cord blood, bone marrow, etc.). Overlays of air or one or more inert gases may also be used within the vessels to improve the storage and stability properties of the compositions described herein. Also included are kits of such compositions, with instructions on how to utilize the compositions in various therapeutic applications, such as *ex vivo* therapeutic applications. With such properties, the compositions of the present invention unexpectedly avoid many of the undesirable characteristics otherwise associated with the use of previously known compositions of prostaglandin EP ligands for *in vitro, ex vivo,* and/or *in vivo* applications.

In certain embodiments, using the improved compositions provided herein to expand the number of bone marrow derived stem cells, such as hematopoietic stem cells, is useful in transplantation and other therapies, particularly for hematologic and oncologic diseases. According to such methods, HSC numbers may be increased *in vitro, ex vivo,* and/or *in vivo.* Such methods are useful because a significant number of autologous donor transplants contain insufficient stem cells, or HSCs. Likewise, patients are often unable to find histocompatible donors, emphasizing the need for improved compositions for expansion of HSCs prior to transplantation. The ability to increase HSC numbers *in vitro* or *ex vivo* allows the collection of fewer cells from donors, thereby reducing the time and discomfort associated with bone marrow/peripheral stem cell harvesting, and increasing the pool of willing HSC donors.

The compositions of the present invention may also be useful to expand the use of cord blood and other cell transplant therapies. Umbilical cord blood banks are widespread and contain a broad variety of donor cells (*i.e.,* histocompatibility types), but for practical purposes the cord blood from these banks is often limited to use in children. This limitation exists mainly because adult stem cell therapies require relatively substantial numbers of stem cells, and most cord blood samples contain inadequate stem cell numbers for this purpose. Thus, compositions and methods to increase stem cell numbers in cord blood would allow this rich and varied source of HSCs to be useful in adult stem cell therapies, thereby expanding the availability and usefulness of allogeneic transplantation for adults. The compositions of the present invention provide these and other uses and advantages that will be apparent to persons skilled in the art.

Unless defined otherwise, all technical and scientific terms used herein have the same meaning as commonly understood by those of ordinary skill in the art to which the invention belongs. Although any methods and materials similar or equivalent to those described herein can be used in the practice or testing of the present invention, preferred methods and materials are described. For the purposes of the present invention, the following terms are defined below. All references referred to herein are incorporated by reference in their entirety.

The articles "a" and "an" are used herein to refer to one or to more than one (*i.e.* to at least one) of the grammatical object of the article. By way of example, "an element" means one element or more than one element. By "about" is meant a quantity, level, value, number, frequency, percentage, dimension, size, amount, weight or length that varies by as much as 30, 25, 20, 25, 10, 9, 8, 7, 6, 5, 4, 3, 2 or 1 % to a reference quantity, level, value, number, frequency, percentage, dimension, size, amount, weight or length.

An "agent," as used herein, relates generally to any chemical compound, peptide, antibody, antibody fragment, carbohydrate, fatty acid, or other molecule that binds to or functionally interacts with a prostaglandin EP receptor, and/or that increase or enhances cyclic AMP levels (*e.g*., intracellular levels) or activity in a cell. In certain embodiments, an "agent" may have one or both of these characteristics, such as by interacting with an EP receptor and increasing cyclic AMP levels or activity. In certain embodiments, an agent may only have one of these activities, such as by increasing cAMP levels independent of an EP receptor. Embodiments include free acid or free base forms of such agents, as well as pharmaceutical salts *(e.g.,* lithium, sodium, etc.) and ester derivatives of such agents, which may be optionally modified at any suitable position (*e.g*., the oxygen atom of an available hydroxyl or carboxyl group) *(see, e.g.,* HANDBOOK OF PHARMACEUTICAL SALTS: PROPERTIES, SELECTION, AND USE. P. Heinrich Stahl & Camille G. Wermuth, Editors, Wiley-VCH; 1st Edition (June 15, 2002), herein incorporated by reference in its entirety).

Throughout this specification, unless the context requires otherwise, the words "comprise," "comprises," and "comprising" will be understood to imply the inclusion of a stated step or element or group of steps or elements but not the exclusion of any other step or element or group of steps or elements.

The term "biological material" refers to any living biological material, including cells, tissues, organs, and/or organisms, and any combination thereof. It is contemplated that the methods of the present invention may be practiced on a part of an organism (such as in cells, in tissue, and/or in one or more organs), whether that part remains within the organism or is removed from the organism, or on the whole organism. Moreover, it is contemplated that in the context of cells and tissues, both homogenous and heterogeneous cell populations may be the subject of embodiments of the invention. The term "*in vivo* biological matter" refers to biological matter that is *in vivo, i.e.,* still within or attached to an organism. Moreover, the term "biological matter" will be understood as synonymous with the term "biological material." In certain embodiments, it is contemplated that one or more cells, tissues, or organs are separate from an organism. The terms "isolated," and *"ex vivo"* are used generally to describe such biological material. It is contemplated that the methods of the present invention may be practiced on *in vivo,* isolated, and/or *ex vivo* biological material.

By "consisting of' is meant including, and limited to, whatever follows the phrase "consisting of." Thus, the phrase "consisting of' indicates that the listed elements are required or mandatory, and that no other elements may be present.

By "consisting essentially of' is meant including any elements listed after the phrase, and limited to other elements that do not significantly interfere with or contribute to the activity or action specified in the disclosure for the listed elements. Thus, the phrase "consisting essentially of' indicates that the listed elements are required or mandatory, but that other elements are optional and may or may not be present depending upon whether or not they significantly affect (*e.g*., statistically) the activity or action of the listed elements.

In reference to chemicals, such as organic chemicals, "analog" or "derivative" relates to a chemical molecule that is similar to another chemical substance in structure and function, often differing structurally by a single element or group, but may differ by differ by modification of more than one group *(e.g.,* 2, 3, or 4 groups) if it retains the same function as the parental chemical. Such modifications are routine to persons skilled in the art, and include, for example, additional or substituted chemical moieties, such as esters or amides of an acid, protecting groups such as a benzyl group for an alcohol or thiol, and tert-butoxylcarbonyl groups for an amine. Also included are modifications to alkyl side chains, such as alkyl substitutions (e.g., methyl, dimethyl, ethyl, etc.), modifications to the level of saturation or unsaturation of side chains, and the addition of modified groups such as substituted phenyl and phenoxy. Derivatives may also include conjugates, such as biotin or avidin moieties, enzymes such as horseradish peroxidase and the like, and including radio-labeled, bioluminescent, chemoluminescent, or fluorescent moieties. Also, moieties may be added to the agents described herein to alter their pharmacokinetic properties, such as to increase half-life *in vivo* or *ex vivo,* or to increase their cell penetration properties, among other desirable properties. Also included are prodrugs, which are known to enhance numerous desirable qualities of pharmaceuticals (*e.g*., solubility, bioavailability, manufacturing, etc.) *(see, e.g.,* WO/2006/047476 for exemplary EP agonist prodrugs, which is incorporated by reference for its disclosure of such agonists).

In reference to polypeptides, "derivative" relates to a polypeptide that has been derived from the basic sequence by modification, for example by conjugation or complexing with other chemical moieties (*e.g*., pegylation) or by post-translational modification techniques as would be understood in the art. The term "derivative" also includes within its scope alterations that have been made to a parent sequence including additions, deletions, and/or substitutions that provide for functionally equivalent or functionally improved molecules.

By "enzyme conditions" it is meant that any necessary conditions are available in an environment *(i.e.,* such factors as temperature, pH, lack of inhibiting substances, etc.) that will permit the agent to function. Enzyme reactive conditions can be either *in vitro* or *ex vivo,* such as in a test tube, or *in vivo,* such as within a subject.

As used herein, the terms "function" and "functional" and the like refer generally to a biological or enzymatic function.

By "isolated" is meant material that is substantially or essentially free from components that normally accompany it in its native state. For example, an "isolated ligand," "isolated HSC" or an "isolated polypeptide" and the like, as used herein, refer to *in vitro* or *ex vivo* isolation and/or purification/enrichment of a ligand *(e.g.,* prostaglandin) or polypeptide molecule from its natural cellular environment, and from association with other components of the cell or tissue, *i.e.,* it is not significantly associated with *in vivo* substances.

"Polypeptide," "polypeptide fragment," "peptide" and "protein" are used interchangeably herein to refer to a polymer of amino acid residues and to variants and synthetic analogues of the same. Thus, these terms apply to amino acid polymers in which one or more amino acid residues are synthetic non-naturally occurring amino acids, such as a chemical analogue of a corresponding naturally occurring amino acid, as well as to naturally-occurring amino acid polymers. In certain aspects, polypeptides may include enzymatic polypeptides, or "enzymes," which typically catalyze (*i*.*e.*, increase the rate of) various chemical reactions.

By "enhance" or "enhancing," or "increase" or "increasing" refers generally to the ability of one or agents or compositions to produce or cause a greater physiological response *(i.e.,* downstream effects) in a cell, as compared to the response caused by either no agent or a control molecule/composition. A measurable physiological response may include greater cell growth or expansion, among others apparent from the understanding in the art and the description herein. An "increased" or "enhanced" amount is typically a "statistically significant" amount, and may include an increase that is 1.1, 1.2, 2, 3, 4, 5, 6, 7, 8, 9, 10, 15, 20, 30 or more times *(e.g.,* 500, 1000 times) (including all integers and decimal points in between and above 1), *e.g.,* 1.5, 1.6, 1.7. 1.8, etc.) the amount produced by no agent (the absence of an agent) or a control composition.

A "cyclic AMP (cAMP) enhancer," described in greater detail below, refers generally to an agent that produces or causes a greater amount of cAMP in a cell, or a greater amount of cAMP activity in a cell, or any other relevant component of a cAMP related signal transduction pathway, or a measurable downstream physiological response or effect of a cAMP signaling pathway, as compared to no agent or a control molecule/composition. Measurable downstream effects may include greater stem cell growth or expansion, among others apparent from the understanding in the art and the description herein. cAMP enhancers may include "agonists," which typically bind to a receptor or other molecule of a cell and trigger a response by the cell, and "antagonists," which typically act against and block/inhibit an action, such as by blocking the degradation of cAMP (*e.g*., blocking a phosphodiesterase). Also included are cAMP analogs.

Examples of cAMP enhancers include, but are not limited to, dibutyryl cAMP (DBcAMP), phorbol ester, forskolin, sclareline, 8-bromo-cAMP, cholera toxin (CTx), aminophylline, 2,4 dinitrophenol (DNP), norepinephrine, epinephrine, isoproterenol, isobutylmethylxanthine (IBMX), caffeine, theophylline (dimethylxanthine), dopamine, rolipram, iloprost, prostaglandin E₁, prostaglandin E₂, pituitary adenylate cyclase activating polypeptide (PACAP), and vasoactive intestinal polypeptide (VIP).

A "ligand to a prostaglandin EP receptor," described in greater detail below, refers generally to any naturally-occurring or synthetic chemical molecule or polypeptide that binds to and/or interacts with an EP receptor, typically to activate or increase one or more of the downstream signaling pathways associated with a prostaglandin EP receptor, as described herein and known in the art. Included within this definition are prostaglandins (PGEs), such as "PGE₂," as well as "analogs" or "derivatives" thereof. Prostaglandins relate generally to hormone like molecules that are derived from fatty acids containing 20 carbon atoms, including a 5-carbon ring, as described herein and known in the art.

Examples of PGE₂ "analogs" or "derivatives" include, but are not limited to, 16,16-dimethyl PGE₂, 16-16 dimethyl PGE₂ p-(p-acetamidobenzamido) phenyl ester, 11-deoxy-16,16-dimethyl PGE₂, 9-deoxy-9-methylene-16, 16-dimethyl PGE₂, 9-deoxy-9-methylene PGE₂, 9-keto Fluprostenol, 5-trans PGE₂, 17-phenyl-omega-trinor PGE₂, PGE₂ serinol amide, PGE₂ methyl ester, 16-phenyl tetranor PGE₂, 15(S)-15-methyl PGE₂, 15(R)-15-methyl PGE₂, 8-iso-15-keto PGE₂, 8-iso PGE₂ isopropyl ester, 20-hydroxy PGE₂, 11-deoxy PGE₁, nocloprost, sulprostone, butaprost, 15-keto PGE₂, and 19 (R) hydroxyy PGE₂. Also included are PG analogs or derivatives having a similar structure to PGE₂ that are substituted with halogen at the 9-position (*see, e.g.,* WO 2001/12596, herein incorporated by reference in its entirety), as well as 2-decarboxy-2-phosphinico prostaglandin derivatives, such as those described in U.S. Publication No. 2006/0247214, herein incorporated by reference in its entirety).

Ligands include "agonists," which typically bind to a receptor of a cell and trigger a response by the cell, and which often mimic the action of a naturally occurring substance. In certain embodiments, ligands include "antagonists," which typically act against and block/inhibit an action.

A "prostaglandin EP receptor" relates generally to any one of the four subtypes of G-protein couple receptors that are within the EP receptor family, referred to as EP₁, EP₂, EP₃, and EP₄.

The recitation "non-PGE₂-based ligand" refers to molecules that are relatively structurally unrelated to a PGE₂ ligand, *i.e.,* are not necessary a PGE₂ analog or derivative, but are otherwise capable of binding to and stimulating an EP receptor. Individual non-PGE₂-based ligands may be capable of stimulating any one or more of the EP receptors, and, thus, may be characterized as an EP₁ agonist, an EP₂ agonist, an EP₃ agonist, and/or an EP₄ agonist, including any combinations thereof. Examples of non-PGE₂-based EP₁ agonists include ONO-DI-004 and ONO-8713. Examples of non-PGE₂-based EP₂ agonists include CAY10399, ONO_8815Ly, ONO-AE1-259, and CP-533,536. Examples of non-PGE₂-based EP₃ agonists include AE5-599, MB28767, GR 63799X, ONO-NT012, and ONO-AE-248. Examples of non-PGE₂-based EP₄ agonists include ONO-4819, APS-999 Na, AH23848, and ONO-AE1-329. Additional examples of non-PGE₂-based EP₂ agonists include the carbazoles and fluorenes disclosed in WO 2007/071456, herein incorporated by reference for its disclosure of such agents. Additional examples of non-PGE₂-based EP₄ agonists can be found in WO/2000/038663; U.S. Patent No. 6,747,037; and U.S. Patent No. 6,610,719, each of which are incorporated by reference for their disclosure of such agonists.

In certain embodiments, the "concentration" of an agent in a composition of the present invention may be defined by standard units of concentration, such as molarity, *e.g.*, µM, mM, etc. For instance, in certain embodiments the agent may be present in a suitable organic solvent at a final concentration of about 100nM to about 10mM, or about 1mM to about 10mM, or about 100nM to about 1µM, or about 10mM.

In certain embodiments, the concentration may be defined by its biological activity, including, for example, the "IC₅₀, "EC₅₀," and/or "EC₉₀" of a selected agent. The "IC₅₀," or half maximal inhibitory concentration, refers to a measure of the effectiveness of am agent or composition in inhibiting a biological or biochemical function. This quantitative measure indicates how much of a particular agent is required to inhibit a given biological process or component of a process by half, and is commonly used as a measure of "antagonist" drug potency in pharmacological research. Sometimes, this measure may be converted to the pIC₅₀ scale (-log IC₅₀), in which higher values indicate exponentially greater potency. According to the Food and Drug Administration (FDA), IC₅₀ represents the concentration of a drug that is required for 50% inhibition *in vitro.*

The term "EC₅₀," or half maximal effective concentration, refers to the concentration of an agent or composition that induces a response halfway between the baseline and maximum, and is commonly used as a measure of drug potency for agonists/stimulators. The EC₅₀ of a graded dose response curve represents the concentration of an agent or composition at which 50% of its maximal effect is observed. Likewise, the EC₅₀ of a quantal dose response curve represents the concentration of an agent or composition at which 50% of the population exhibits a response. EC₅₀ also represents the plasma concentration required for obtaining 50% of a maximum effect *in vivo.* Similarly, the "EC₉₀" refers to the concentration of an agent or composition at which 90% of its maximal effect is observed. The "EC₉₀" can be calculated from the "EC₅₀" and the Hill slope, or it can be determined from the data directly, using routine knowledge in the art.

In certain embodiments, the final concentration of an EP ligand when dispensed into an *ex vivo* treatment vessel may be at least comparable to the IC₅₀ or EC₅₀ of its EP receptor binding affinity and/or EP receptor activation. As another example, in certain embodiments the concentration may be at least about 10 times the EC₉₀ of its EP receptor binding affinity and/or EP receptor activation. In certain embodiments, the EP ligand shows an EC₉₀ that is no less than 5% of the EC₉₀ of the ligand prior to its introduction into an endotoxin-free vessel. In certain embodiments, such as wherein the ligand is 16,16-dimethyl PGE₂, the ligand shows an EC₅₀ that is within about 10% of the EC₅₀ of the same batch of the ligand prior to its introduction into an endotoxin-free vessel, as measured, for example, by a colony-forming unit (CFU) assay. CFU assays are known in the art.

EP receptor binding affinity and EP receptor activation can be measured according to routine techniques in the art and described herein. For instance, a detailed preparation for measuring affinity for the mouse EP receptors is outlined in Kirimaya et al. (Br J Pharmacol. 122:217-24, 2007), herein incorporated by reference in its entirety. Likewise, a prep measuring affinity for the human receptors is described in WO 2005/061449 *(see, e.g.,* page 71), herein incorporated by reference in its entirety. Binding affinities may be measured for any of the individual 4 receptor subtypes EP₁, EP₂, EP₃ and EP₄, including combinations thereof, as well as for other anti-targets, such as prostanoid receptors DP and IP. In certain embodiments, binding affinities may be measured for EP₂ and/or EP₄ receptors, and spot checked for the others.

EP receptor activation and/or EP ligand functional assays are also known in the art. For example, activation of the EP₂ and EP₄ receptors increases intracellular cAMP levels, which may be monitored according to standard functional assays. As a particular example, WO 2005/061449, herein incorporated by reference in its entirety, describes a general procedure to monitor functional efficacy at the EP₂ receptor (*see* page 73). Similar assays are available for the EP₄ and other EP receptors.

The recitation "organic solvent" or "suitable organic solvent" relates generally to carbon containing liquids or gases that dissolve a solid, liquid, or gaseous solute, resulting in a solution. A "suitable" organic solvent is one that is appropriate for *ex vivo* administration to, or incubation with, mammalian cells, and may also be appropriate for *in vivo* administration to a subject, such as by having minimal toxicity or other inhibitory effects under *ex vivo* conditions *(e.g.,* cell culture) or *in vivo* at a selected concentration for the time of incubation or administration. A suitable organic solvent should also be appropriate for storage stability and handling of the agents described herein. Examples of suitable organic solvents include, but are not limited to, dimethyl sulfoxide (DMSO), N,N-dimethylformamide (DMF), dimethoxyethane (DME), and dimethylacetamide, including mixtures or combinations thereof. In certain embodiments, a composition or organic solvent is "substantially free" of methyl acetate, meaning that there should be no more than trace amounts of methyl acetate in the composition or solvent, and preferably undetectable amounts (e.g., as measured by high pressure liquid chromatography (HPLC), gas chromatography (GC), etc.).

The recitation "endotoxin free" relates generally to compositions, solvents, and/or vessels that contain at most trace amounts *(i.e.,* amounts having no adverse physiological effects to a subject) of endotoxin, and preferably undetectable amounts of endotoxin. Endotoxins are toxins associated with certain bacteria, typically gram-negative bacteria, although endotoxins may be found in gram-positive bacteria, such as *Listeria monocytogenes.* The most prevalent endotoxins are lipopolysaccharides (LPS) or lipo-oligo-saccharides (LOS) found in the outer membrane of various Gram-negative bacteria, and which represent a central pathogenic feature in the ability of these bacteria to cause disease. Small amounts of endotoxin in humans may produce fever, a lowering of the blood pressure, and activation of inflammation and coagulation, among other adverse physiological effects.

Therefore, in pharmaceutical production, it is often desirable to remove most or all traces of endotoxin from drug product containers, because even small amounts may cause adverse effects in humans. A depyrogenation oven may be used for this purpose, as temperatures in excess of 300°C are typically required to break down most endotoxins. For instance, based on primary packaging material such as syringes or vials, the combination of a glass temperature of 250°C and a holding time of 30 minutes is often sufficient to achieve a 3 log reduction in endotoxin levels.

Endotoxins can be detected using routine techniques known in the art. For example, the Limulus Ameobocyte Lysate assay, which utilizes blood from the horseshoe crab, is a very sensitive assay for detecting presence of endotoxin. In this test, very low levels of LPS can cause detectable coagulation of the limulus lysate due a powerful enzymatic cascade that amplifies this reaction.

In certain embodiments, the "purity" of any given agent in a composition may be specifically defined. For instance, certain compositions may comprise an agent that is at least 90, 91, 92, 93, 94, 95, 96, 97, 98, 99, or 100% pure, including all decimals in between, as measured by high pressure liquid chromatography (HPLC), a well-known form of column chromatography used frequently in biochemistry and analytical chemistry to separate, identify, and quantify compounds.

An "inert gas," as used herein, relates to any gas that is not reactive with elements. Certain embodiments may include an "inert gas" in the composition, such as an inert gas overlay. Inert gases may be utilized, for instance, to increase storage stability of an agent of the present invention, such as by reducing oxygen content. Examples of inert gases include nitrogen, argon, helium, neon, krypton, xenon, radon, flue gas, and sometimes carbon dioxide, as well as mixtures thereof.

Similarly, certain embodiments may employ an "air overlay," which refers generally to an air space between an agent-containing solvent and the lid or other part of a vessel. In certain aspects, the air overlay may include straight air, as known in the art, or air of reduced oxygen content, such as air mixed with an inert gas.

A "vessel" relates generally to any suitable container for storing and/or administering a composition of the present invention, and is typically endotoxin free, as described herein and known in the art. A vessel may be suitable for any type of storage conditions, such as room temperature conditions and/or cryogenic storage conditions. A "vessel" may also be suitable for containing cells, such as HSCs, in addition to a composition of the present invention, such as during *ex vivo* therapeutic applications (*e.g*., incubating the cells with, or exposing them to, the composition within a selected vessel, such as a PE or polyvinyl chloride (PVC) bag). Examples of vessels include, but are not limited to, bags (e.g., medical grade PE and/or PVC bags, etc.), pouches, capsules, vials, tubes (*e.g*., microcentrifuge tubes, EPPENDORF TUBES®, FALCON® conical tubes, etc.), bottles, dishes (*e.g.,* Petri dishes, etc.), implant devices (*e.g*., collagen sponges, etc.), flasks, bioreactors, and syringes. In certain embodiments, the vessel is a single use vessel. Also, in certain embodiments, the vessel may be coated with one or more of the agents of the invention (*e.g*., PGE₂, or an analog). Embodiments of the present invention also include trays of such vessels, and kits comprising vessels or trays of such vessels.

The recitation "good manufacturing practice (GMP)" refers generally to the control and management of manufacturing, and quality control testing, of foods, pharmaceutical products, and medical devices. GMP does not necessarily rely on sampling, but instead relies on documentation of every aspect of the process, activities, and operations involved with drug and medical device manufacture. If the documentation showing how the product was made and tested (which enables traceability and, in the event of future problems, recall from the market) is not correct and in order, then the product does not meet the required specification and is considered *contaminated* (*i.e., adulterated* in the US). Additionally, GMP typically requires that all manufacturing and testing equipment has been qualified as suitable for use, and that all operational methodologies and procedures (*e.g*., manufacturing, cleaning, and analytical testing) utilized in the drug manufacturing process have been validated according to predetermined specifications to demonstrate that they can perform their purported function(s). In the US, the phrase "current good manufacturing practice" appears in 501(B) of the 1938 Food, Drug, and Cosmetic Act (21 U.S.C. § 351).

"Hematopoietic stem cells (HSCs)" relate generally to either pluripotent or multipotent "stem cells" that give rise to the blood cell types, including myeloid (*e.g*., monocytes and macrophages, neutrophils, basophils, eosinophils, erythrocytes, megakaryocytes/platelets, dendritic cells), and lymphoid lineages (*e.g*., T-cells, B-cells, NK-cells), and others known in the art. "Stem cells" are typically defined by their ability to form multiple cell types (*i.e*., multipotency) and their ability to self-renew. In certain embodiments, however, oligopotent and unipotent progenitors may be included.

"Hematopoiesis" refers generally to the process of cellular differentiation or formation of particular, specialized blood cells from an HSC. During development, hematopoiesis translocates from the fetal liver to the bone marrow, which then remains the site of hematopoiesis throughout adulthood. Once established in the bone marrow, HSCs are not distributed randomly throughout the bone cavity. Rather, HSCs are typically found in close proximity to the endosteal surfaces. The more mature stem cells increase in number as the distance from the bone surface increases. Finally, as the central longitudinal axis is approached, terminal differentiation occurs.

Hematopoietic tissues typically contain cells with long-term and short-term regeneration capacities, as well as committed multipotent, oligopotent, and unipotent progenitors. Recently, long-term transplantation experiments point toward a clonal diversity model of hematopoietic stem cells. In such models, the HSC compartment consists of a fixed number of different types of HSC, each with epigenetically preprogrammed behavior. HSCs are believed to constitute about 1:10,000 of cells in myeloid tissue.

HSCs may be obtained according to known techniques in the art. For instance, HSCs may be found in the bone marrow of adults, which includes femurs, hip, ribs, sternum, and other bones. HSCs may be obtained directly by removal from the hip using a needle and syringe, or from the blood, often following pre-treatment with cytokines, such as G-CSF (granulocyte colony-stimulating factors), that induce cells to be released from the bone marrow compartment. Other sources for clinical and scientific use include umbilical cord blood, placenta, and mobilized peripheral blood. For experimental purposes, fetal liver, fetal spleen, and AGM (Aorta-gonad-mesonephros) of animals are also useful sources of HSCs.

HSCs may be identified according to certain phenotypic or genotypic markers. For example, HSCs may be identified by their small size, lack of lineage (lin) markers, low staining (side population) with vital dyes such as rhodamine 123 (rhodamine^{DULL}, also called rho^{lo}) or Hoechst 33342, and presence of various antigenic markers on their surface, many of which belong to the cluster of differentiation series (*e.g.,* CD34, CD38, CD90, CD133, CD105, CD45, and c-kit, the receptor for stem cell factor). HSCs are mainly negative for the markers that are typically used to detect lineage commitment, and, thus, are often referred to as lin(-) cells. Most human HSCs may be characterized as CD34⁺, CD59⁺, Thyl/CD90⁺, CD38^{lo/-}, C-kit/CD117⁺, and lin(-). However, not all stem cells are covered by these combinations, as certain HSCs are CD34⁻/CD38⁻. Also some studies suggest that earliest stem cells may lack c-kit on the cell surface. For human HSCs, CD133 may represent an early marker, as both CD34⁺ and CD34⁻ HSCs have been shown to be CD133⁺.

For purification of lin(-) HSCs by flow cytometry, or FACS, an array of mature blood-lineage marker antibodies may be used to deplete the lin(+) cells or late multipotent progenitors (MPP), including, for example, antibodies to CD13 and CD33 for human myeloid cells, CD71 for human erythroid cells, CD19 for human B cells, CD61 for human megakaryocytic cells, Mac-1 (CD11b/CD18) for monocytes, Gr-1 for Granulocytes, I17Ra, CD3, CD4, CD5, and CD8 for T cells, among others known in the art. Other purification methods are known in the art, such as those methods that use the particular signature of the 'signaling lymphocyte activation molecules' (SLAM) family of cell surface molecules.

HSCs, whether from cord blood, bone marrow, peripheral blood, or other source, may be grown or expanded in any suitable, commercially available or custom defined medium, with or without serum, as desired *(see, e.g.,* Hartshorn et al., Cell Technology for Cell Products, pages 221-224, R. Smith, Editor; Springer Netherlands, 2007, herein incorporated by reference in its entirety). For instance, in certain embodiments, serum free medium may utilize albumin and/or transferrin, which have been shown to be useful for the growth and expansion of CD34+ cells in serum free medium. Also, cytokines may be included, such as Flt-3 ligand, stem cell factor (SCF), and thrombopoietin (TPO), among others. HSCs may also be grown in vessels such as bioreactors *(see, e.g.,* Liu et al., Journal of Biotechnology 124:592-601, 2006, herein incorporated by reference in its entirety). A suitable medium for *ex vivo* expansion of HSCs may also comprise HSC supporting cells, such as stromal cells (*e.g*., lymphoreticular stromal cells), which can be derived, for instance, from the disaggregation of lymphoid tissue, and which have been show to support the *in vitro, ex vivo,* and *in vivo* maintenance, growth, and differentiation of HSCs, as well as their progeny.

"Hematopoietic stem cell (HSC) growth or expansion" can be measured *in vitro* or *in vivo* according to routine techniques known in the art. For example, WO 2008/073748, herein incorporated by references for these methods, describes methods for measuring *in vivo* and *in vitro* expansion of HSCs, and for distinguishing between the growth/expansion of HSCs and the growth/expansion of other cells in a potentially heterogeneous population (*e.g*., bone marrow), such as intermediate progenitor cells. The administering or incubation step that results in the growth or expansion can occur *in vivo, ex vivo,* or *in vitro,* though in certain embodiments, the administration or incubation occurs during *ex vivo* treatment of HSCs. An unexpanded population of HSCs and HSC supporting cells refers to an HSC population prior to or in the substantial absence of exposure to a prostaglandin, prostaglandin receptor agonist, or cAMP enhancer, as described herein.

"Cord blood" or "umbilical cord blood" relates generally to the relatively small amount of blood (up to about 180mL) from a newborn baby that returns to the neonatal circulation if the umbilical cord is not prematurely clamped. Cord blood is rich in HSCs, and may be harvested and stored for later use according to techniques known in the art (*see, e.g.,* U.S. Patent Nos. 7,147,626 and 7,131,958, herein incorporated by reference for such methodologies). Also, if the umbilical cord is ultimately not clamped, a physiological clamping occurs upon interaction with cold air, wherein the internal gelatinous substance, called Wharton's jelly, swells around the umbilical artery and veins. Nonetheless, Wharton's jelly can still serve as a source of stem cells.

As noted above, *"ex vivo"* refers to generally to activities that take place outside an organism, such as experimentation or measurements done in or on living tissue in an artificial environment outside the organism, preferably with minimum alteration of the natural conditions. Most commonly, *"ex vivo"* procedures involve living cells or tissues taken from an organism and cultured in a laboratory apparatus, usually under sterile conditions, and typically for a few hours or up to about 24 hours, but including up to 48 or 72 hours, depending on the circumstances. In certain embodiments, such tissues or cells can be collected and frozen, and later thawed for *ex vivo* treatment. Tissue culture experiments or procedures lasting longer than a few days using living cells or tissue are typically considered to be *"in vitro,"* though in certain embodiments, this term can be used interchangeably with *ex vivo.*

The recitations *"ex vivo* administration," *"ex vivo* treatment," or *"ex vivo* therapeutic use," relate generally to medical procedures in which one or more organs, cells, or tissues are obtained from a living or recently deceased subject, optionally purified/enriched, exposed to a treatment or procedure to expand the stem cells (*e.g.,* an *ex vivo* administration step that involves incubating the cells with a composition of the present invention to enhance expansion of desirable cells, such as HSCs), and then administered to the same or different living subject after that optional treatment or procedure.

Such *ex vivo* therapeutic applications may also include an optional *in vivo* treatment or procedural step, such as by administering a composition of the invention one or more times to the living subject after administration of the organ, cells, or tissue. Both local and systemic administration are contemplated for these embodiments, according to well-known techniques in the art. The amount of agent administered to a subject will depend on the characteristics of that subject, such as general health, age, sex, body weight, and tolerance to drugs, as well as the degree, severity, and type of reaction to the drug and/or cell transplant.

A "subject," as used herein, includes any animal that exhibits a symptom that can be treated with an agent or composition or device of the invention, or can be treated with HSCs or cord blood that have been treated *ex vivo* with an agent or composition of the invention. A "subject" also includes anyone who is a candidate for stem cell transplant or bone marrow transplantation, such as during the course of treatment for a malignant disease or a component of gene therapy. Subjects may also include individuals or animals that donate stem cells or bone marrow for allogeneic transplantation. In certain embodiments, a subject may have undergone irradiation therapy or chemotherapy, such as during various cancer treatments. Suitable subjects (*e.g*., patients) include laboratory animals (such as mouse, rat, rabbit, or guinea pig), farm animals, and domestic animals or pets (such as a cat or dog). Non-human primates and, preferably, human patients, are included. Typical subjects include animals that exhibit aberrant amounts (lower or higher amounts than a "normal" or "healthy" subject) of one or more physiological activities that can be modulated by an agent or a stem cell or marrow transplant.

"Treatment" or "treating," as used herein, includes any desirable effect on the symptoms or pathology of a disease or pathological condition, and may include even minimal reductions in one or more measurable markers of the disease or condition being treated. "Treatment" does not necessarily indicate complete eradication or cure of the disease or condition, or associated symptoms thereof. The subject receiving this treatment is any animal in need, including primates, in particular humans, and other mammals such as equines, cattle, swine and sheep; and poultry and pets in general.

The terms "pathogenic" or "pathological condition," as used herein, relate to a disease, abnormal condition or injury of a mammalian cell, tissue, or organ, typically those that may benefit from cell transplant-based therapies. Such pathological conditions include, for example, hyperproliferative and unregulated neoplastic cell growth, degenerative conditions, inflammatory diseases, autoimmune diseases and infectious diseases. Pathological conditions characterized by excessive or unregulated cell growth include, for example, hyperplasia, cancer, autoimmune disease and infectious disease. Hyperplastic and cancer cells proliferate in an unregulated manner, causing destruction of tissues and organs. Specific examples of hyperplasias include benign prostatic hyperplasia and endometrial hyperplasia. Specific examples of cancer include prostate, breast, ovary, lung, uterus, brain and skin cancers.

Abnormal cellular growth can also result from infectious diseases in which foreign organisms cause excessive growth. Infectious diseases can also damage tissues, such as by causing apoptosis and/or necrosis, and further cause excessive biological responses that contribute to the pathological condition, such as high fevers. The growth of cells infected by a pathogen, such as a virus or intracellular bacteria, is abnormal due to the alteration of the normal condition of a cell resulting from the presence of a foreign organism. Specific examples of infectious diseases include DNA and RNA viral diseases, bacterial diseases, and parasitic diseases. Similarly, the growth of cells mediating autoimmune and inflammatory diseases are aberrantly regulated which results in, for example, the continued proliferation and activation of immune mechanisms with the destruction of tissues and organs.

By specific mention of the above categories of pathological conditions, those skilled in the art will understand that such terms include all classes and types of these pathological conditions, certain examples of which are described elsewhere herein. For example, the term cancer is intended to include all known cancers, whether characterized as malignant, benign, soft tissue or solid tumor. Similarly, the terms infectious diseases, degenerative diseases, autoimmune diseases and inflammatory diseases are intended to include all classes and types of these pathological conditions.

### Cyclic AMP (cAMP) Enhancers

The cAMP enhancers of the present invention typically increase or maintain the intracellular levels and/or activity of cAMP. Most generally, cyclic adenosine monophosphate (cAMP, cyclic AMP or 3'-5'-cyclic adenosine monophosphate) acts as an important secondary messenger in many biological processes. Secondary messenger systems relate to methods of cellular signaling, whereby a diffusible signaling molecule is rapidly produced/secreted upon a certain activation signal, which can then activate effector proteins within the cell to exert a cellular response. For instance, among other responses, cAMP signaling transfers the effects of hormones like glucagon and adrenaline, which otherwise cannot pass through the cell membrane. cAMP also regulates the passage of Ca²⁺ through ion channels.

cAMP is synthesized from ATP by adenylyl cyclase, the latter being localized to cell membranes. Adenylyl cyclase may be activated by a range of signaling molecules, such as through the activation of adenylyl cyclase stimulatory G (Gₛ)-coupled receptors. For instance, liver adenylyl cyclase responds strongly to glucagon, and muscle adenylyl cyclase responds strongly to adrenaline. Therefore, cAMP and its associated kinases function in several biochemical processes, including the regulation of glycogen, sugar, and lipid metabolism.

The cAMP dependent pathway, also known as the adenylyl cyclase pathway, is a G protein-coupled receptor triggered signaling cascade used in cell communication. G protein-coupled receptors (GPCRs) are a large family of integral membrane proteins that respond to a variety of extracellular stimuli. Each GPCR binds to and is activated by a specific ligand stimulus that ranges in size from small molecule catecholamines, lipids, or neurotransmitters to large protein hormones. When a GPCR is activated by its extracellular ligand, it undergoes a conformational change that is transmitted to an attached intracellular heterotrimeric G protein complex. In response, the Gₛ alpha subunit of the stimulated G protein complex exchanges GDP for GTP, and is then released from the complex to signal the next step in the pathway.

In a cAMP dependent pathway, the activated Gₛ alpha subunit typically binds to and activates adenylyl cyclase, which in turn catalyzes the conversion of ATP into cAMP, as noted above. Increases in concentration of the second messenger cAMP may lead to the activation of cyclic nucleotide-gated ion channels, exchange proteins activated by cAMP (EPAC) such as RAPGEF3, and/or protein kinase A (PKA). Specificity of signaling between a GPCR and its ultimate molecular target through a cAMP dependent pathway may be achieved through formation of a multi protein complex, including the GPCR, adenylyl cyclase, and the effector protein.

As noted above, cyclic AMP activates protein kinase A (PKA, also known as cAMP-dependent protein kinase). PKA is normally inactive as a tetrameric holoenzyme, consisting of 2 catalytic and 2 regulatory units (C₂R₂), with the regulatory units blocking the catalytic centers of the catalytic units. Cyclic AMP binds to specific locations on the regulatory units of PKA, dissociates the regulatory and catalytic subunits, and thereby activates the catalytic units, enabling them to phosphorylate substrate proteins. Not all protein kinases respond to cAMP, as several types of protein kinases are not cAMP dependent, including, for example, protein kinase C.

The active subunits of PKA may catalyze the transfer of phosphate from ATP to specific serine or threonine residues of protein substrates. The phosphorylated protein kinases may act directly on ion channels in the cell, or may activate or inhibit other enzymes. PKA also phosphorylates specific proteins that bind to promoter regions of DNA, causing increased expression of specific genes. Further downstream effects depend on the various roles of PKA, which may differ based on the type of cell. For instance, activated PKA may phosphorylate a number of other proteins, including, for example, proteins that convert glycogen into glucose, proteins that promote muscle contraction in heart leading to an increase in heart rate, and transcription factors that regulate gene expression.

cAMP activity may be negatively regulated by a variety of mechanisms. For instance, the Gₛ alpha subunit slowly catalyzes the hydrolysis of GTP to GDP, which in turn deactivates the Gₛ protein, thereby shutting off the cAMP pathway. The cAMP pathway may also be deactivated downstream by directly inhibiting adenylyl cyclase or by dephosphorylating the proteins phosphorylated by PKA. Adenylyl cyclase, and thus cAMP production, may be inhibited by agonists of adenylyl cyclase inhibitory G (Gᵢ)-protein coupled receptors. cAMP decomposition into AMP is catalyzed by the enzyme phosphodiesterase, which may also act as a negative regulator of cAMP signaling.

Examples of molecules that inhibit cAMP pathway include, for example cAMP phosphodiesterase, which dephosphorylates cAMP into AMP, reducing the cAMP levels; Gᵢ protein, which inhibits adenylyl cyclase, thereby reducing cAMP levels; and pertussis toxin, which decrease cAMP levels.

The cAMP enhancers of the present invention are typically capable of activating the cAMP pathway at any of the stages in that pathway, or may prevent the negative regulation (e.g., degradation) of cAMP, and include chemicals, polypeptides, antibodies, and other molecules having such functional effects. Exemplary molecules or agents that activate cAMP pathway may include, for instance, cholera toxin, which increases cAMP levels; forskolin, a diterpine natural product that activates adenylyl cyclase; caffeine and theophylline, which inhibit cAMP phosphodiesterase, leading to an activation of G proteins that then activate the cAMP pathway; and bucladesine (dibutyryl cAMP, DBcAMP), which is also a phosphodiesterase inhibitor.

Examples of cAMP enhancers include, but are not limited to, dibutyryl cAMP (DBcAMP), phorbol ester, forskolin, sclareline, 8-bromo-cAMP, cholera toxin (CTx), aminophylline, 2,4 dinitrophenol (DNP), norepinephrine, epinephrine, isoproterenol, isobutylmethylxanthine (IBMX), caffeine, theophylline (dimethylxanthine), dopamine, rolipram, iloprost, prostaglandin E₁, prostaglandin E₂, pituitary adenylate cyclase activating polypeptide (PACAP), and vasoactive intestinal polypeptide (VIP), among others known in the art. As exemplified above, examples of cAMP enhancers also include cAMP and analogs of cAMP, such sp-5,6-DCl-BIMPS (BIMPS), among others.

cAMP is implicated in the growth and/or survival of hematopoietic stem cells in culture (*see, e.g.,* Negrotto et al., Experimental Hematology 34:1420-1428, 2006, herein incorporated by reference in its entirety). For instance, it was observed that two different cAMP analogs, such as dibutyryl-cAMP and BIMPS, promote survival of human umbilical cord-derived CD34+ cells by suppressing apoptosis induced by either nitric oxide (NO) or serum deprivation. Involvement of PKA and PI3K pathway was demonstrated by the ability of their specific inhibitors Rp-cAMP and Wortmannin or LY294002, respectively, to reverse the antiapoptotic effect of BIMPS. While thrombopoietin (TPO), granulocyte colony-stimulating factor (G-CSF), or stem cell factor (SCF) did not increase cAMP levels, the antiapoptotic activity exerted by these growth factors was blocked by inhibition of the adenylate cyclase and synergized by BIMPS. Thus, cyclic AMP analogs suppress the decreased colony formation in cells exposed to NO or serum deprivation, showing that cAMP appears to be not only a key pathway controlling CD34+ survival, but also a mediator of TPO, G-CSF, and SCF-mediated cytoprotection.

Likewise, activation of cAMP, such as by injection of isoproterenol (which stimulates adenylyl cyclase) or dibutyryl cyclic adenosine 3',5'-monophosphate shortly after marrow cell graft, almost immediately triggers the transplanted stem cells into entering S phase by inducing DNA synthesis (*see, e.g.,* Necas et al., Cell Proliferation, 9:223-230, 2008, herein incorporated by reference in its entirety).

Therefore, compositions of the invention that comprise cAMP enhancers may be utilized to increase the survival and/or expansion of hematopoietic stem cells in culture (*e.g., in vitro* or *ex vivo)* and/or *in vivo,* such as prior to or subsequent to bone marrow, stem cell, and/or HSC-based transplant procedures, thereby increasing the overall number of HSCs administered to a recipient subject.

### Prostaglandin EP Receptors and Receptor Ligands

There are a number of different prostaglandin receptors on various cell types. These prostaglandin receptors represent a sub-family of the cell surface seven-transmembrane receptors referred to as G-protein-coupled receptors. G protein-coupled receptors (GPCR), comprise a large protein family of transmembrane receptors that detect molecules outside the cell and activate intracellular signal transduction pathways, thereby activating downstream cellular responses. G protein-coupled receptors are found only in eukaryotes, including yeast, plants, choanoflagellates, and animals. Generally, the ligands that bind and activate these receptors include light-sensitive compounds, odors, pheromones, hormones, and neurotransmitters, and vary in size from small molecules to peptides to large proteins. GPCRs are involved in many diseases, and, thus, represent the direct or indirect target of many modern medicines.

Of the many prostaglandin receptors, there are currently four prostaglandin EP receptors: EP₁, EP₂, EP₃, and EP₄. When activated by a suitable ligand, or agonist, such as a prostaglandin or analog thereof, these prostaglandin receptors initiate a variety of downstream biological functions. For example, these four receptors are coupled either to Ca²⁺ mobilization (EP₁ and EP₃) or to the stimulation of adenylyl cyclase (EP₂ and EP₄)

Prostaglandins are lipid compounds derived enzymatically from fatty acids. Typically, prostaglandins contain 20 carbon atoms, including a 5-carbon ring. These molecules mediate a variety of strong physiological effects, and although they are technically hormones, they are rarely classified as such. For instance, as autocrine and paracrine lipid mediators, prostaglandins act upon platelets, endothelium, uterine and mast cells, among other cells.

Generally, prostaglandins are produced by all nucleated cells except lymphocytes. Prostaglandins are typically synthesized in a cell from the essential fatty acids (EFAs). For example, prostaglandins may be produced following the sequential oxidation of arachidonic acid (AA), gamma-linolenic acid (GLA, via DGLA), or eicosapentaenoic acid (EPA) by cyclooxygenases (COX-1 and COX-2) and terminal prostaglandin synthases. COX-1 is believed to be responsible for the baseline levels of prostaglandins, and COX-2 mainly produces prostaglandins through stimulation.

From these EFA starting molecules, phospholipase-A₂ catalyzes the formation of an intermediate molecule, which then enters either the cyclooxygenase pathway or the lipoxygenase pathway to form, respectively, either prostaglandin and thromboxane, or leukotriene. The cyclooxygenase pathway typically produces thromboxane, prostacyclin, and prostaglandins D, E and F. The lipoxygenase pathway is typically active in leukocytes and macrophages, and synthesizes leukotrienes.

Prostaglandin E2 (PGE₂) is a primary product of arachidonic acid metabolism, and is typically synthesized via the cyclooxygenase and prostaglandin synthase pathways. For instance, PGE₂ may be generated from the action of prostaglandin E synthases on prostaglandin H₂ (PGH₂). Several prostaglandin E synthases have been identified, such as microsomal prostaglandin E synthase-1, which represents a key enzyme in the formation of PGE₂.

PGE₂ and its analogs activate the various EP receptors to initiate numerous downstream activities. For instance, among other effects, activation of the EP₁ receptor by PGE₂ stimulates bronchoconstriction and gastro-intestinal tract smooth muscle contraction. Activation of the EP₂ receptor by PGE₂ stimulates bronchodilation, gastro-intestinal tract smooth muscle relaxation, and vasodilation. Activation of the EP₃ receptor by PGE₂ stimulates decreased gastric acid secretion, increased gastric mucous secretion, uterus contraction (during pregnancy), gastro-intestinal smooth muscle contraction, lipolysis inhibition, and increases autonomic neurotransmitters, among other effects. In addition, PGE₂ is released by blood vessel walls in response to infection or inflammation, and may act on the brain to induce fever. Thus, PGE₂ has pyrogenic effects, and may also lead to hyperalgesia

PGE₂, its analogs, and related prostaglandins have many clinical uses. For instance, among other uses, synthetic prostaglandins are used to induce childbirth (parturition) or abortion (*e.g*., PGE₂ or PGF₂, with or without mifepristone, a progesterone antagonist), to prevent closure of patent ductus arteriosus in newborns with particular cyanotic heart defects (*e.g.,* PGE₁), to prevent and treat peptic ulcers (*e.g.,* PGE), as a vasodilator in severe Raynaud's phenomenon or ischemia of a limb, in treating pulmonary hypertension, in treatment of glaucoma (*e.g*., as a bimatoprost ophthalmic solution, a synthetic prostamide analog with ocular hypotensive activity), and to treat erectile dysfunction or in penile rehabilitation following surgery (*e.g*., PGE₁ as alprostadil).

Prostaglandins are also implicated in other clinically relevant physiological processes. For example, the role of the prostaglandin synthesis pathway, particularly the rate-limiting enzymatic step catalyzed by cyclooxygenase, to colorectal carcinogenesis and development of novel anticolorectal cancer therapy is well established. The predominant PG species in benign and malignant colorectal tumors is PGE₂. As noted above, PGE₂ acts via four EP receptors termed EP₁ to EP₄. Thus, EP receptors have been identified as potential targets for treatment and/or prevention of colorectal cancer (*See, e.g.,* Hull et al., Mol Cancer Ther. 3:1031-9, 2004, herein incorporated by reference in its entirety).

Also, PGE₂ is thought to be a principal fever mediator *(see, e.g.,* Oka et al., Brain Research 98:256-262, 2003, herein incorporated by reference in its entirety). For instance, ONO-DI-004, an EP1 receptor agonist, has been shown to increase the core temperature (*T_{c}*) in a dose-dependent manner with a time course similar to PGE₂-induced hyperthermia. Likewise, ONO-AE-248 (20 nmol), an EP₃ receptor agonist, also increased the *T*_{c}. In contrast, ONO-AE1-329, an EP4 receptor agonist, decreased the *T*_{c}, and ONO-AE1-259-01, an EP₂ receptor agonist, was shown to not change the *T*_{c}*.* Thus, the EP₁, EP₃, and EP₄ receptors all may contribute to the thermoregulatory response to PGE₂, but each may have a different role.

Prostaglandins are also implicated in the regulation of cellular proliferation and survival. For example, PGE₂ inhibits the mitogenesis of hepatic stellate cells, which are central to liver fibrosis (*see, e.g.,* Hui et al., Prostaglandins Leukot Essent Fatty Acids. 71:329-33, 2004, herein incorporated by reference in its entirety). PGE₂ and EP2-selective agonists have been shown to produce dose-dependent inhibitory effects on PDGF-stimulated proliferation. In contrast, neither EP₁, EP₃, nor EP₄-selective agonists show any inhibitory effect. Adenylyl cyclase inhibitors strongly blunt the inhibition of DNA synthesis elicited by PGE₂ and the EP2 agonist, showing the role of cAMP in this process. Thus, activation of the PGE EP₂ receptor has an antiproliferative effect on hepatic stellate cells that may be mediated by cyclic AMP-related signal transduction pathways. In this regard, certain of the compositions of the present invention may be used to reduce the proliferation of hepatic stellate cells in therapies for liver fibrosis.

PGE₂ and its analogs also play an essential role in dendritic cell (DC) migration and survival (*see, e.g.,* Vassiliou et al., The Journal of Immunology, 173: 6955-6964, 2004, herein incorporated by reference in its entirety). Studies have demonstrated that PGE₂ protects DC *ex vivo* or *in vitro* against apoptosis induced by withdrawal of growth factors or ceramide. It has been shown that DC matured in conditions that inhibit endogenous PGE₂ release are highly susceptible to apoptosis and that the addition of exogenous PGE₂ re-establishes the more resistant phenotype. This antiapoptotic effect is mediated through EP₂/EP₄ receptors and likely involves the PI3K → Akt pathway. In particular, PGE₂ leads to increased phosphorylation of Akt, protection against mitochondrial membrane compromise, and decreased caspase 3 activity. Also, macroarray data indicate that PGE₂ leads to the down-regulation of a number of proapoptotic molecules, such as BAD, several caspases, and granzyme B. *In vivo,* higher numbers of immature and antigen-loaded CFSE-labeled DC are present in the draining lymph nodes of mice inoculated with PGE₂ receptor agonists, compared with animals treated with ibuprofen or controls injected with PBS. Therefore, by acting as an endogenous antiapoptotic factor for DC, compositions comprising PGE₂-based agonists may be used *ex vivo* and/or *in vivo* to increase the survival of *ex vivo* antigen-loaded DC following administration to a subject in need thereof.

PGE₂ also regulates hematopoietic stem cell homeostasis in vertebrates (*see, e.g.,* North et al., Nature 447:1007-1011, 2007; and Broxmeyer, Cell 1:135-136, 2007, both of which are incorporated by reference in their entirety). Hematopoietic stem cell (HSC) homeostasis is tightly controlled by growth factors, signaling molecules and transcription factors. Definitive HSCs derived during embryogenesis in the aorta-gonad-mesonephros region subsequently colonize fetal and adult hematopoietic organs. In this context, it has been shown that that chemicals that enhance PGE₂ synthesis increased HSC numbers, and those that block prostaglandin synthesis decreased stem cell numbers. Also, it has been shown that the cyclooxygenases responsible for PGE₂ synthesis were also required for HSC formation. In addition, a stable derivative of PGE2 as been shown to improve kidney marrow recovery following irradiation injury in the adult zebrafish. In murine embryonic stem cell differentiation assays, PGE₂ has been shown to cause amplification of multipotent progenitors. Furthermore, *ex vivo* exposure to stabilized PGE₂ enhances spleen colony forming units at day 12 post transplant and increases the frequency of long-term repopulating HSCs present in murine bone marrow after limiting dilution competitive transplantation. Thus, the conserved role for PGE2 in the regulation of vertebrate HSC homeostasis indicates that modulation of the prostaglandin pathway may facilitate expansion of HSC number for therapeutic purposes.

Therefore, according to certain of the compositions and methods provided herein, bioactive PGE₂ and their analogs or derivatives can accelerate recovery of the hematopoietic system following chemotherapy or irradiation, and/or to promote growth and expansion of hematopoietic stem cells during *ex vivo* treatments, such as prior to, during, and/or after transplant procedures *(see, e.g.,* WO 2008/073748, herein incorporated by reference in its entirety). Likewise, also according to the methods provided herein, *ex vivo* expansion of HSCs in the presence of PGE₂ or its analogs prior to stem cell transplantation can improve reconstitution of hematopoiesis and immune function after transplant *(see, e.g.,* Lord et al., Cell Cycle 6:3054-7, 2007, herein incorporated by reference in its entirety).

Therefore, embodiments of the present invention include methods of stimulating hematopoietic stem cell (HSC) growth, homing, or expansion, as well as the growth or expansion of other stem-like cells (e.g., multi-potent cells, pluripotent cells, etc.) comprising transferring the improved compositions that comprise either a cAMP enhancer or EP ligand, as well as a suitable organic solvent, into a vessel that is suitable for *ex vivo* treatment conditions, wherein the vessel comprises cord blood or HSCs in suitable medium, optionally incubating the cells for a time sufficient to stimulate growth or expansion of the HSCs, and thereby stimulating HSC growth or expansion. Alternatively, such methods may be accomplished by transferring HSCs (or other source of HSCs, such as cord blood or bone marrow) into a suitable vessel (*e.g.,* PE bag, tube, etc.) that already contains the compositions of the present invention, such as a coated vessel. Such variations for practicing the claimed methods will be apparent to persons skilled in the art based on the description provided herein.

The improved compositions of the present may be prepared according to techniques described herein and understood in the art. For instance, in certain embodiments, an agent selected from a cyclic AMP (cAMP) enhancer and a ligand to a prostaglandin EP receptor may have already been dissolved in an organic solvent, such as methyl acetate, that may be otherwise unsuitable for use in *ex vivo* therapeutic uses, and/or may contribute to undesirable storage or handling properties of such agent. In this regard, certain commercial sources of EP ligands are provided in methyl acetate.

Thus, to arrive at the compositions of the present invention from such a source of an agent, methods of preparing a composition suitable for *ex vivo* administration to mammalian cells may comprise the steps of reducing the volume of a first composition in a vessel that comprises methyl acetate and an agent of the present invention, to create a second composition, wherein the second composition is substantially free of the methyl acetate; and then adding an organic solvent to the second composition in the vessel, wherein the organic solvent is not methyl acetate and is suitable for *ex vivo* administration to mammalian cells, to obtain a final composition that has a suitable concentration of the agent, as described herein (*e.g.,* 10mM). In certain embodiments, reducing the volume of the first composition may comprise evaporating the methyl acetate. Also, the second solvent is typically a suitable organic solvent, as described herein (e.g., DMSO, DMF, DME, etc., including combinations or mixtures thereof). One or more solvents may be combined at certain ratios. For instance, a mixture of two solvents may be combined at a ratio of 9.5:0.5, 9:1, 8:2, 7:3, 6:4, 5:5, etc., including all integers and decimal points.

In certain embodiments, methods of preparing a composition of the present invention may further comprise the step of transferring the composition from the initial or first vessel noted above to a second vessel, wherein the second vessel is endotoxin free and is suitable for storage or *ex vivo* administration of the composition. In this regard, the second vessel may be considered more suitable for storage, handling, shipping, and/or *ex vivo* therapeutic use than the first vessel, the latter of which may have been derived, for instance, from the commercial source of the selected agent. Alternatively, the first vessel may have been considered suitable in all respects.

In particular, certain of these methods may involve transferring the composition from the first vessel to a second vessel, wherein the second vessel is 2ml vial with a teflon cap that is endotoxin free and is suitable for storage or *ex vivo* administration of the composition, wherein the agent is 16,16 dimethyl PGE₂ at final concentration of about 10mM, wherein the organic solvent is dimethyl sulfoxide (DMSO) that is substantially free of methyl acetate, and wherein there is an air overlay in the vial. Preferably, the entire composition, including the vessel and the solvent, is sterile and endotoxin-free.

In certain embodiments, the second or other vessel may comprise cells, such as HSCs or cord blood. Accordingly, these and other embodiments may involve transferring the composition from the first or initial vessel, or even the second vessel noted in the previous paragraph, to another vessel (*i.e*., second or third vessel) that is suitable for *ex vivo* treatment conditions and that comprises cord blood, or another source of human cells in a suitable medium. Alternatively, the source of human cells may be transferred to the first or second vessel that already contains the composition, such as a PE bag or tube, and which is already suitable for *ex vivo* treatment or incubation conditions.

In certain embodiments, the cord blood or source of human cells may comprise HSCs, whether as a relatively heterogeneous population (*e.g*., cord blood, bone marrow, etc), or as a relatively purified or enriched homogenous population, either population being prepared as described herein and known in the art. In certain embodiments, the final concentration of the suitable organic solvent upon incubation with the cells may be less than about 0.01% to less than about 1% of the total volume of the suitable medium, including all decimal points below and in between *(e.g.,* 0.005, 0.03, 0.05, 0.08, etc.), or may be characterized by its IC₅₀, EC₅₀, or EC₉₀, as described herein.

Embodiments also include kits, comprising one or more vessels described herein, or trays of such vessels, wherein the vessels comprise a composition of the present invention. Also, such kits may further include instructions on use of the composition for *ex vivo* administration to mammalian cells. The instructions may include guidance on the isolation, enrichment, purification, and expansion of stem cells, such as HSCs, and may include directions on the optimization of dosing, timing of exposure to the compositions of the invention, timing on expansion, optimal cell numbers for expansion and subsequent administration, culture conditions and/or media, and /or final preparation for administration to a subject, among other variables expected to be important in *ex vivo* therapeutic uses. In certain embodiments, the *ex vivo* administration to mammalian cells or HSCs comprises *ex vivo* therapeutic use in humans.

Embodiments of the present invention are further described by reference to the following non-limiting examples.

### EXAMPLE 1

### EX VIVO TREATMENT OF HEMATOPOIETIC STEM CELLS (HSCS) PRIOR TO TRANSPLANT

This example shows the manner in which the agents of the present invention may be used to enhance the *ex vivo* growth of HSCs prior to transplant. Bone marrow cells containing HSCs are obtained directly by removal from the hip of a donor subject using a needle and syringe. The heterogeneous mixture of bone marrow cells is transferred to two separate tissue culture flasks, and a suitable growth medium is provided.

262 µl of a 10mM solution of 16,16-dimethyl PGE₂ in DMSO is added to the cells in the first tissue culture flask, to a final working concentration of 10 µM of 16,16-dimethyl PGE₂. The same amount of DMSO without agent is added to the second flask as a control, and the cells in both flasks are incubated at 4°C *(e.g.,* in an ice bath) for one hour. The 16,16-dimethyl PGE₂ is at least 90% pure as measured by HPLC, and is produced by good manufacturing practice (GMP).

After incubation of the HSCs in the ice bath, the cells from each flask are administered to separate, but otherwise genetically identical subjects. These subjects are both allogeneic to the donor cells. The subjects are then observed for engraftment of the donor HSCs, using markers that are specific for the donor cells. The subject receiving a transplant of the 16,16-dimethyl PGE₂ treated cells is shown to have a significantly greater number of engrafted HSC donor cells than the subject receiving the control-treated cells.

### EXAMPLE 2

### CELL-BASED ASSAY FOR CAMP ENHANCER ACTIVITY

This example shows an immunoassay that can be used to determine the potency of cAMP enhancer compounds, as reflected by cAMP levels. Specifically, a fluorescence resonance energy transfer time-resolved (FRET) is used to quantitate cAMP levels in cells treated with cAMP enhancer compounds. In this assay, cells are treated with a test compound and then lysed to release cytosolic cAMP. Biotinylated cAMP, a fluorophore-tagged antibody to cAMP, and Europium chelate-streptavidin are added to the cell lysates and allowed to equilibrate, as shown in Figure 1.

In the absence of cytosolic cAMP, such as in a control sample, the α-cAMP antibody binds the biotinylated cAMP, and the biotin moiety binds the Europium chelate through streptavidin. This binding complex allows energy transfer from the Europium chelate to the fluorophore upon excitation with light at a wavelength of about 340 nm. The excited fluorophore then emits a certain, baseline level of light at a characteristic wavelength, which is measured by a fluorometer.

In the presence of cytosolic cAMP, such as after treatment of cells with a cAMP enhancer, the increased levels of cytosolic cAMP displace the Europium chelate-streptavidin/biotinylated cAMP complex from the fluorophore labeled α-cAMP antibody. This process effectively removes Europium from the vicinity of the fluorophore, prevents energy transfer required for fluorophore excitation and emission, and results in reduced signal detection by the fluorometer. Thus, in this assay, the potency of a cAMP enhancer may be measured by the degree of signal reduction in a treated cell as compared to a control.

### EXAMPLE 3

### BIOLOGICAL ASSAY FOR CAMP ACTIVITY

The potency of cAMP enhancer compounds may also be measured according to the effects of elevated cAMP levels on cell function. For example, an increase in the frequency of engraftable hematopoietic stem cells can be measured with a colony forming unit-spleen (CFU-S) assay. In this assay, human bone marrow or cord blood is treated with a cAMP enhancer compound *ex vivo* and then administered intravenously into immunocompromised mice (*e.g*., NOD/SCID mice) that have been sublethally irradiated to suppress endogenous spleen colonization. Twelve to 14 days after administration of the cells, the spleens are removed from the mice, and the cell colonies produced from the spleen-engrafted hematopoietic stem cells are counted. The number of splenic colonies reflects the frequency of engraftable hematopoietic stem cells, which is increased after administration of a cAMP enhancer.

### EXAMPLE 4

### LIQUID CHROMATOGRAPHY ULTRAVIOLET (LC/UV) DETECTION OF 16,16-DIMETHYL PGE₂

16,16-dimethyl PGE₂ and its related substances were separated on a Phenomenex, Synergi Hydro RP 4 µ HPLC column with gradient elution conditions and detected at 205 nm. The assay of 16,16-dimethyl PGE₂ was determined by comparing the 16,16-dimethyl PGE₂ peak area of the test samples with that of a reference standard of known concentration and purity. The levels of the impurities and degradation products were determined from the peak area ratio of each individual impurity to the total peak area of the 16,16-dimethyl PGE₂ and all impurities and degradation products. The identify of 16,16-dimethyl PGE₂ was confirmed by comparing the retention time of the main peak of the test sample against that in the reference standard.

The method was determined and completed using the following chromatographic parameters:
Solvent A - Wather with 0.04% formic acid
Solvent B - Methanol with 0.04% formic acid
LC/UV Gradient 4 (shown in Table 1 below)
Detection at 205 nm
Flow rate 1.0 mL/min
Needle rinse with DMSO/MeOH 1/1 to prevent carryover
Autosampler draw speed at 50 µl/min for viscous solution
Injection volume at 10 µl

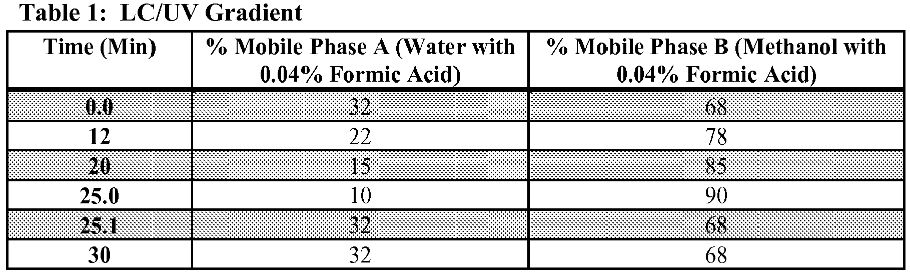

After the method parameters were determined, method qualification was performed for linearity, instrument precision, and limit of qualification. A summary of the qualification results is shown in Table 2 below.

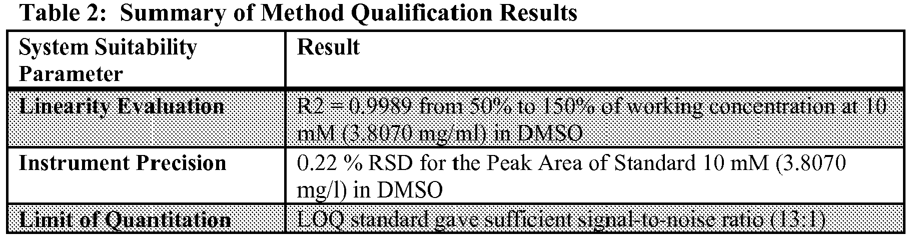

Retention times of 16,16-dimethyl PGE₂ were very consistent between sample and standard injections. Identification of sample based on retention time was also achieved. Using these methods, assay of 16,16-dimethyl PGE₂, 10 mM in DMSO was 98.5%.

### EXAMPLE 5

### STABILITY OF 16,16-DIMETHYL PGE₂ FORMULATION

Stability tests were performed on formulations of 16,16-dimethyl PGE₂ stored at either -80°C or -20°C for up to six months. These clinical formulations were composed of 10mM 16,16-dimethyl PGE₂ in 1 mg/ml DMSO, and were stored in a 2ml Type I clear glass vial with a 13mm West 4432/50 Teflon coated stopper. The results are shown in Tables 3 (-80°C) and 4 (-20°C) below.

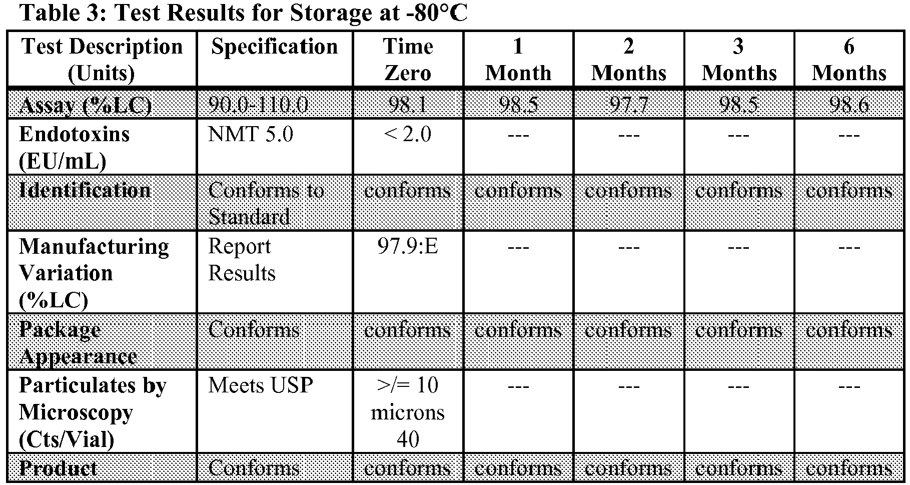

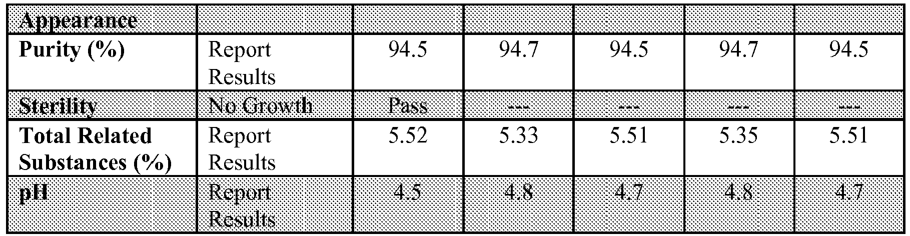

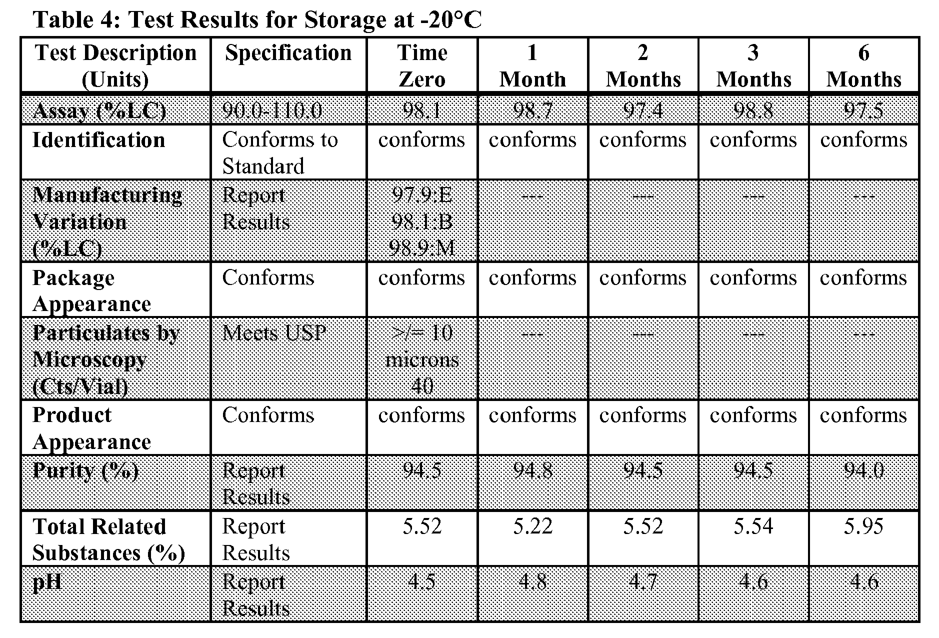

The invention is now defined by the following clauses:

### Clauses

1. A composition comprising an agent selected from a cyclic AMP (cAMP) enhancer and a ligand to a prostaglandin EP receptor, and an organic solvent, wherein the agent and the organic solvent are contained within a sterile and endotoxin free vessel, and wherein the composition is suitable for *ex vivo* administration to human cells.
2. The composition of clause 1, wherein the agent is a cAMP enhancer.
3. The composition of clause 2, wherein the cAMP enhancer is selected from the group consisting of dibutyryl cAMP (DBcAMP), phorbol ester, forskolin, sclareline, 8-bromo-cAMP, cholera toxin (CTx), aminophylline, 2,4 dinitrophenol (DNP), norepinephrine, epinephrine, isoproterenol, isobutylmethylxanthine (IBMX), caffeine, theophylline (dimethylxanthine), dopamine, rolipram, iloprost, prostaglandin E₁, prostaglandin E₂, pituitary adenylate cyclase activating polypeptide (PACAP), and vasoactive intestinal polypeptide (VIP).
4. The composition of clause 1, wherein the agent is a ligand to a prostaglandin receptor.
5. The composition of clause 4, wherein the ligand is a prostaglandin EP receptor agonist.
6. The composition of clause 4, wherein the ligand is prostaglandin E₂ (PGE₂), or an analog thereof.
7. The composition of clause 6, wherein the PGE₂ analog is selected from the group consisting of 16,16-dimethyl PGE₂, 16-16 dimethyl PGE₂ p-(p-acetamidobenzamido) phenyl ester, 11-deoxy-16,16-dimethyl PGE₂, 9-deoxy-9-methylene-16, 16-dimethyl PGE₂, 9-deoxy-9-methylene PGE₂, 9-keto Fluprostenol, 5-trans PGE₂, 17-phenyl-omega-trinor PGE₂, PGE₂ serinol amide, PGE₂ methyl ester, 16-phenyl tetranor PGE₂, 15(S)-15-methyl PGE₂, 15(R)-15-methyl PGE₂, 8-iso-15-keto PGE₂, 8-iso PGE₂ isopropyl ester, 20-hydroxy PGE₂, 11-deoxy PGE₁, nocloprost, sulprostone, butaprost, 15-keto PGE₂, and 19 (R) hydroxyy PGE₂.
8. The composition of clause 7, wherein the PGE₂ analog is 16,16-dimethyl PGE₂.
9. The composition of clause 4, wherein the ligand is a non-PGE₂-based ligand.
10. The composition of clause 9, wherein the non-PGE₂-based ligand is selected from the group consisting of an EP₁ agonist, an EP₂ agonist, an EP₃ agonist, and an EP₄ agonist.
11. The composition of clause 10, wherein the non-PGE₂-based EP₁ agonist is selected from the group consisting of ONO-DI-004 and ONO-8713.
12. The composition of clause 10, wherein the non-PGE₂-based EP₂ agonist is selected from the group consisting of CAY10399, ONO_8815Ly, ONO-AE1-259, and CP-533,536.
13. The composition of clause 10, wherein the non-PGE₂-based EP₃ agonist is selected from the group consisting of AE5-599, MB28767, GR 63799X, ONO-NT012, and ONO-AE-248.
14. The composition of clause 10, wherein the non-PGE₂-based EP₄ agonist is selected from the group consisting of ONO-4819, APS-999 Na, AH23848, and ONO-AE1-329.
15. The composition of clause 1, wherein the agent is present at a concentration of about 100nM to about 10mM.
16. The composition of clause 1, wherein the agent is present at a concentration of about 1mM to about 10mM.
17. The composition of clause 1, wherein the agent is present at a concentration of about 100nM to about 1µM.
18. The composition of clause 1, wherein the agent is present at a concentration of about 10mM.
19. The composition of clause 1, wherein the agent is produced by good manufacturing practice (GMP).
20. The composition of clause 1, wherein the agent is at least 90%, 95%, or 98% pure by high pressure liquid chromatography (HPLC).
21. The composition of clause 4, wherein the prostaglandin EP receptor is selected from EP₁, EP₂, EP₃, and EP₄.
22. The composition of clause 1, wherein the organic solvent is substantially free of methyl acetate.
23. The composition of clause 1, wherein the organic solvent is selected from the group consisting of dimethyl sulfoxide (DMSO), N,N-dimethylformamide (DMF), dimethoxyethane (DME), dimethylacetamide, and combinations thereof.
24. The composition of clause 23, wherein the organic solvent is DMSO.
25. The composition of clause 1, comprising an inert gas in the vessel.
26. The composition of clause 1, comprising an air overlay in the vessel.
27. The composition of clause 1, wherein the vessel is a bag, capsule, vial, tube, dish, or syringe that is suitable for storage of the composition.
28. The composition of clause 1, wherein the vessel is a single use vessel.
29. The composition of clause 1, wherein the vessel is a bag, vial, tube, dish, or syringe that further comprises cord blood or human cells in a suitable medium, and wherein the vessel is suitable for *ex vivo* treatment of the cells.
30. The composition of clause 29, wherein the organic solvent volume is less than about 1% of the total volume of the suitable medium.
31. The composition of clause 29, wherein the organic solvent volume is less than about 0.1% of the total volume of the suitable medium.
32. The composition of clause 29, wherein the human cells comprise hematopoietic stem cells.
33. The composition of clause 1, wherein the agent is 16,16 dimethyl PGE₂ at a final concentration of about 10mM, wherein the organic solvent is dimethyl sulfoxide (DMSO) that is substantially free of methyl acetate, wherein the vessel is a 2ml vial with a teflon coated stopper, and wherein there is an air overlay in the vial.
34. A method of preparing a composition suitable for *ex vivo* administration to human cells, comprising
   (a) reducing the volume of a first composition in an endotoxin free vessel that comprises methyl acetate and an agent selected from a cyclic AMP (cAMP) enhancer and a ligand to a prostaglandin EP receptor, to create a second composition, wherein the second composition is substantially free of the methyl acetate; and
   (b) adding an organic solvent to the second composition in the vessel, wherein the organic solvent is not methyl acetate and is suitable for *ex vivo* administration to human cells,
   thereby preparing the composition suitable for *ex vivo* administration to human cells.
35. The method of clause 34, wherein reducing the volume of the first composition in (a) comprises evaporating the methyl acetate.
36. The method of clause 34, wherein the agent is a cAMP enhancer.
37. The method of clause 36, wherein the cAMP enhancer is selected from the group consisting of dibutyryl cAMP (DBcAMP), phorbol ester, forskolin, sclareline, 8-bromo-cAMP, cholera toxin (CTx), aminophylline, 2,4 dinitrophenol (DNP), norepinephrine, epinephrine, isoproterenol, isobutylmethylxanthine (IBMX), caffeine, theophylline (dimethylxanthine), dopamine, rolipram, iloprost, prostaglandin E₁, prostaglandin E₂, pituitary adenylate cyclase activating polypeptide (PACAP), and vasoactive intestinal polypeptide (VIP).
38. The method of clause 34, wherein the agent is a ligand to a prostaglandin receptor.
39. The method of clause 38, wherein the ligand is a prostaglandin EP receptor agonist.
40. The method of clause 38, wherein the ligand is prostaglandin E₂ (PGE₂), or an analog thereof.
41. The method of clause 40, wherein the PGE₂ analog is selected from the group consisting of 16,16-dimethyl PGE₂, 16-16 dimethyl PGE₂ p-(p-acetamidobenzamido) phenyl ester, 11-deoxy-16,16-dimethyl PGE₂, 9-deoxy-9-methylene-16, 16-dimethyl PGE₂, 9-deoxy-9-methylene PGE₂, 9-keto Fluprostenol, 5-trans PGE₂, 17-phenyl-omega-trinor PGE₂, PGE₂ serinol amide, PGE₂ methyl ester, 16-phenyl tetranor PGE₂, 15(S)-15-methyl PGE₂, 15(R)-15-methyl PGE₂, 8-iso-15-keto PGE₂, 8-iso PGE₂ isopropyl ester, 20-hydroxy PGE₂, 11-deoxy PGE₁, nocloprost, sulprostone, butaprost, 15-keto PGE₂, and 19 (R) hydroxyy PGE₂.
42. The method of clause 1, wherein the PGE₂ analog is 16,16-dimethyl PGE₂.
43. The method of clause 38, wherein the ligand is a non-PGE₂-based ligand.
44. The method of clause 40, wherein the non-PGE₂-based ligand is selected from the group consisting of an EP₁ agonist, an EP₂ agonist, an EP₃ agonist, and an EP₄ agonist.
45. The method of clause 44, wherein the non-PGE₂-based EP₁ agonist is selected from the group consisting of ONO-DI-004 and ONO-8713.
46. The method of clause 44, wherein the non-PGE₂-based EP₂ agonist is selected from the group consisting of CAY10399, ONO_8815Ly, ONO-AE1-259, and CP-533,536.
47. The method of clause 44, wherein the non-PGE₂-based EP₃ agonist is selected from the group consisting of AE5-599, MB28767, GR 63799X, ONO-NT012, and ONO-AE-248.
48. The method of clause 44, wherein the non-PGE₂-based EP₄ agonist is selected from the group consisting of ONO-4819, APS-999 Na, AH23848, and ONO-AE1-329.
49. The method of clause 34, wherein the agent is present in the organic solvent of (b) at a final concentration of about 100nM to about 10mM.
50. The method of clause 34, wherein the agent is present in the organic solvent of (b) at a concentration of about 1mM to about 10mM.
51. The method of clause 34, wherein the agent is present in the organic solvent of (b) at a concentration of about 100nM to about 1µM.
52. The method of clause 34, wherein the agent is present in the organic solvent of (b) at a concentration of about 10mM.
53. The method of clause 34, wherein the agent is produced by good manufacturing practice (GMP).
54. The method of clause 34, wherein the agent is at least 90%, 95%, or 98% pure by high pressure liquid chromatography (HPLC).
55. The method of clause 34, wherein the prostaglandin EP receptor is selected from EP₁, EP₂, EP₃, and EP₄.
56. The method of clause 34, wherein the organic solvent of (b) is selected from the group consisting of dimethyl sulfoxide (DMSO), dimethylformamide (DMF), dimethoxyethane (DME), dimethylacetamide, and combinations thereof.
57. The method of clause 56, wherein the organic solvent is DMSO.
58. The method of clause 34, wherein the vessel is a bag, vial, tube, dish, or syringe that is sterile and endotoxin free and is suitable for storage or *ex vivo* administration of the composition.
59. The method of clause 34, wherein the agent is 16,16 dimethyl PGE₂ at final concentration of about 10mM, wherein the organic solvent of (b) is dimethyl sulfoxide (DMSO) that is substantially free of methyl acetate, wherein the vessel is a 2ml vial with a teflon cap, and wherein there is an air overlay in the vial.
60. The method of clause 34, further comprising
   (c) transferring the composition from the vessel to a second vessel, wherein the second vessel is endotoxin free and is suitable for storage or *ex vivo* administration of the composition.
61. The method of clause 60, wherein the second vessel is a bag, capsule, vial, tube, dish, or syringe.
62. The method of clause 60, wherein the second vessel is a single use vessel.
63. The method of clause 34, further comprising
   (c) transferring the composition from the vessel to a second vessel, wherein the second vessel is 2ml vial with a teflon cap that is endotoxin free and is suitable for storage or *ex vivo* administration of the composition, wherein the agent is 16,16 dimethyl PGE₂ at final concentration of about 10mM, wherein the organic solvent of (b) is dimethyl sulfoxide (DMSO) that is substantially free of methyl acetate, and wherein there is an air overlay in the vial.
64. The method of clause 34, further comprising
   (c) transferring the composition from the vessel to a second vessel, wherein the second vessel is suitable for *ex vivo* treatment conditions and comprises cord blood.
65. The method of clause 34, further comprising
   (c) transferring the composition to a second vessel, wherein the second vessel is suitable for *ex vivo* treatment conditions and comprises human cells in a suitable medium.
66. The method of clause 64 or clause 65, wherein the organic solvent volume is less than about 1% of the total volume of the suitable medium.
67. The method of clause 64 or clause 65, wherein the organic solvent volume is less than about 0.1% of the total volume of the suitable medium.
68. The method of clause 65, wherein the human cells comprise hematopoietic stem cells (HSCs).
69. A method of stimulating hematopoietic stem cell (HSC) growth or expansion, comprising transferring the composition of clause 1 to a second vessel that is suitable for *ex vivo* treatment conditions, wherein the second vessel comprises cord blood or HSCs in suitable medium, thereby stimulating HSC growth or expansion.
70. The method of clause 69, further comprising administering the HSCs to a subject.
71. A kit, comprising a composition of clause 1 and instructions on use of the composition for *ex vivo* administration to human cells.
72. The kit of clause 71, wherein the *ex vivo* administration to human cells comprises *ex vivo* therapeutic use in humans.

## Claims

1. A composition for use in *ex vivo* cell transplant-based therapies comprising:
(a) a ligand to a prostaglandin EP receptor selected from:
(i) prostaglandin E2 (PGE2); or
(ii) a PGE2 analog selected from the group consisting of 16,16-dimethyl PGE2, 16-16 dimethyl PGE2 p-(p- acetamidobenzamido) phenyl ester, 11-deoxy- 16,16-dimethyl PGE2, 9-deoxy-9-methylene-16, 16-dimethyl PGE2, 9-deoxy-9-methylene PGE2, 9-keto Fluprostenol, 5-trans PGE2, 17-phenyl- omega-trinor PGE2, PGE2 serinol amide, PGE2 methyl ester, 16-phenyl tetranor PGE2, 15 (S)-15-methyl PGE2, 15(R)-15-methyl PGE2, 8-iso-15-keto PGE2, 8-iso PGE2 isopropyl ester, 20-hydroxy PGE2, 11-deoxy PGE1, nocloprost, sulprostone, butaprost, 15-keto PGE2, and 19(R) hydroxyPGE2; and
(b) an organic solvent selected from the group consisting of dimethyl sulfoxide (DMSO), N,N-dimethylformamide (DMF), dimethoxyethane (DME), and dimethylacetamide, or combinations thereof.

2. The composition for use in *ex vivo* cell transplant-based therapies of claim 1, wherein the ligand to a prostaglandin EP receptor is 16,16-dimethyl PGE2.

3. The composition for use in *ex vivo* cell transplant-based therapies of claims 1 or 2, wherein the ligand to a prostaglandin EP receptor is at least 90% pure as measured by high pressure liquid chromatography.

4. The composition for use in *ex vivo* cell transplant-based therapies of any one of claims 1 to 3, wherein the ligand to a prostaglandin EP receptor is present in the solvent at a concentration of:
(a) about 100 nM to about 10 mM;
(b) about 1 mM to about 10 mM;
(c) about 100 nM to about 1µM; or
(d) about 10 mM.

5. The composition for use in *ex vivo* cell transplant-based therapies of any one of claims 1-4, wherein the composition is substantially free of methyl acetate.

6. The composition for use in *ex vivo* cell transplant-based therapies of any one of claims 1-5, wherein the composition is contained within a sterile and endotoxin free vessel, and the composition is suitable for *ex vivo* administration to human cells.

7. The composition for use in *ex vivo* cell transplant-based therapies of claim 6, wherein the ligand to a prostaglandin EP receptor is 16,16-dimethyl PGE2 and the ligand shows an EC50 that is within 10% of the EC50 of the same batch of the ligand prior to its introduction into the sterile and endotoxin free vessel.

8. The composition for use in *ex vivo* cell transplant-based therapies of any one of claims 1-6 further comprising human cells in a suitable medium.

9. The composition for use in *ex vivo* cell transplant-based therapies of claim 8, wherein the organic solvent volume is less than about 1% of the total volume of the suitable medium.

10. The composition for use in *ex vivo* cell transplant-based therapies of claim 8, wherein the organic solvent volume is less than about 0.1% of the total volume of the suitable medium.

11. The composition for use in *ex vivo* cell transplant-based therapies of any one of claims 6-7, wherein the vessel is a single-use vessel selected from a bag, capsule, vial, tube, dish, or syringe.

12. The composition for use in *ex vivo* cell transplant-based therapies of any one of claims 8-11, wherein the human cells are hematopoietic stem cells.

13. The composition for use in *ex vivo* cell transplant-based therapies of any one of claims 8-12, wherein the cells are CD34+.
